# FASCICULE DE BREVET EUROPEEN

(11) **EP 3 429 696 B1**
(45) Date de publication et mention de la délivrance du brevet: **05.05.2021**
(21) Numéro de dépôt: 17710011.2
(22) Date de dépôt: 15.03.2017
(51) Int. Cl.: A61Q 19/08, A61K 8/9789, A61K 8/60, A61K 8/64, A23L 33/105, A23L 33/18, A23G 1/30, C12N 9/42, C12N 9/24

(54) **HYDROLYSAT PEPTIDIQUE ET OSIDIQUE DES FÈVES DE CACAO, COMPOSITIONS COSMETIQUES LE COMPRENANT ET LEURS UTILISATIONS COSMÉTIQUES**
PEPTID UND SACCHARIDHYDROLYSAT VON KAKAOBOHNEN, KOSMETISCHE ZUSAMMENSETZUNGEN DAMIT UND KOSMETISCHE VERWENDUNGEN DAVON
PEPTIDE AND SACCHARIDE HYDROLYSATE OF COCOA BEANS, COSMETIC COMPOSITIONS CONTAINING SAME, AND COSMETIC USES OF SAME

(30) Priorité: 16.03.2016 FR 1600441
(43) Date de publication de la demande: 23.01.2019
(73) Titulaire: ISP Investments LLC, Wilmington, DE 19805 (US); Jafer Enterprises R&D, S.L. (Sociedad Unipersonal), 08403 Granollers (Barcelona) (ES)
(72) Inventeur: COQUET, Corinne, 06620 Cipieres (FR); GONDRAN, Catherine, 83440 CALLIAN (FR); IMBERT, Isabelle, 06400 Cannes (FR); MANTELIN, Joel, 06400 Cannes (FR); DOMLOGE, Nouha, 06650 Opio (FR); GARNIER, Sébastien, 06650 Le Rouret (FR); BORSOTTO, Jérémie, Tilleuls 6 06580 Pegomas (FR); CICCHETTI, Esmeralda, 40260 CASTETS (FR); LABARRADE, Florian, 06600 Antibes (FR)
(74) Mandataire: Axe PI
(86) Numéro de dépôt international: PCT/EP2017/056084
(87) Numéro de publication internationale: WO 2017/157998

(56) Documents cités:
- EP-A1- 2 322 041
- EP-A1- 2 517 567
- WO-A1-2008/059064
- WO-A2-2008/015341
- PATRICIA MARTORELL ET AL: "A Cocoa Peptide Protects Caenorhabditis elegans from Oxidative Stress and [beta]-Amyloid Peptide Toxicity", PLOS ONE, vol. 8, no. 5, 13 mai 2013 (2013-05-13), pages e63283-e63283, XP055250509, DOI: 10.1371/journal.pone.0063283
- BAHAREH SARMADI ET AL: "Antioxidant and angiotensin converting enzyme (ACE) inhibitory activities of cocoa (L.) autolysates", FOOD RESEARCH INTERNATIONAL, ELSEVIER APPLIED SCIENCE, BARKING, GB, vol. 44, no. 1, 8 octobre 2010 (2010-10-08), pages 290-296, XP028134794, ISSN: 0963-9969, DOI: 10.1016/J.FOODRES.2010.10.017 [extrait le 2010-10-17]
- DATABASE WPI Week 201282 1 octobre 2012 (2012-10-01) Thomson Scientific, London, GB; AN 2012-Q97780 XP002760983, & CN 102 687 838 A (GUANGZHOU FLOWER FLAVOURS&FRAGRANCES C) 26 septembre 2012 (2012-09-26)

## Description

L'invention concerne un hydrolysat peptidique et osidique des fèves de *Theobroma cacao L* (cacao), son procédé de préparation, des compositions cosmétiques le comprenant et leurs utilisations cosmétiques notamment pour la protection cutanée vis-à-vis de la lumière bleue.

La lumière bleue représente une partie de la lumière visible se situant entre 400 et 495 nm. C'est la partie la plus énergétique du spectre visible. La plus grande partie de la lumière bleue reçue provient du soleil. Dans notre environnement intérieur, nous sommes de plus en plus exposés à la lumière bleue artificielle provenant de LEDs (Light-Emitting Diodes), présents dans les éclairages, les écrans de smartphones, de tablettes, d'ordinateurs et de télévision.

Les photons de la lumière visible sont captés par les photorécepteurs présents sur les cellules de la rétine, initiant le mécanisme de photo-transduction. Ces photorécepteurs appelés opsines sont couplés aux protéines G, et contiennent un chromophore, le 11-cis rétinal, qui subit une réaction de photo-isomérisation en all-trans rétinal, entrainant une cascade de transduction du signal.

La perception de la lumière agit sur l'organisme selon deux voies : la voie optique primaire qui comprend les effets visuels, et la voie rétino-hypothalamique qui est suivie d'effets biologiques tels que la sécrétion de mélatonine, la régulation de la température corporelle, l'expression des gènes circadiens.

Des études ont été menées sur les dommages engendrés par la lumière bleue sur les cellules de la rétine. L'exposition à la lumière visible produit des espèces oxygénées réactives dans les cellules épithéliales de la rétine, créant des dommages de l'ADN mitochondrial et pouvant entrainer l'apoptose de ces cellules2,3. Ces altérations ont pu être reliées au développement de la dégénérescence maculaire liée à l'âge, au cours de laquelle se produit une accumulation de lipofuscine dans les cellules de la rétine4.

De plus, il a récemment été démontré que la lumière bleue induisait l'agrégation des opsines dans des cellules rétiniennes murines, conduisant à des dommages cellulaires5.

Un autre effet de la lumière bleue au travers de l'utilisation d'appareils tels que des tablettes tactiles a été rapporté. L'utilisation de ce type d'appareils avant l'endormissement modifie le taux de mélatonine et conduit à un décalage dans le rythme circadien6. Pour contrer les effets délétères de la lumière bleue, plusieurs marques d'optique ou produisant des appareils contenant des LEDs ont développé des verres ou des écrans bloquant les longueurs d'ondes correspondant au bleu.

La lumière bleue a également des effets sur la peau. Il a été montré sur des kératinocytes et des cellules endothéliales de peau humaine que la lumière bleue exerçait un effet anti-prolifératif et favorisait la différenciation des kératinocytes7. De plus, elle supprime la maturation des cellules dendritiques8. Ces propriétés ont conduit à l'utilisation de la lumière bleue dans le traitement des maladies cutanées hyper-prolifératives et/ou inflammatoires telles que le psoriasis, la dermatite atopique ou l'eczéma.

De plus, l'exposition à la lumière bleue de fragments de peau dont la fonction barrière a été préalablement altérée a montré un retard dans la récupération de la barrière cutanée, notamment en ce qui concerne les lipides sécrétés à l'interface entre le stratum corneum et le stratum granulosum. Ce résultat n'a pas été observé avec d'autres longueurs d'onde du spectre visible, ce qui suggère une activité spécifique de la lumière bleue en relation avec l'homéostasie de la barrière cutanée 1.

En ce qui concerne le traitement de l'acné, l'exposition à la lumière bleue crée une photo-excitation de la porphyrine bactérienne de Propionibacterium acnes, conduisant à l'élimination de la bactérie9.

Très récemment, l'expression des récepteurs opsines OPN1-SW, OPN2, OPN3 et OPN5 a été identifiée dans les kératinocytes et les mélanocytes, suggérant leur rôle dans la photo-transduction au niveau des cellules de la peau10. Les signaux qui découlent de l'activation des opsines dans les cellules de la peau génèrent des effets non-visuels incluant la régulation des gènes circadiens 11.

Considérant les effets néfastes que peut produire la lumière bleue sur la peau, il apparaissait important de pouvoir mettre au point une composition cosmétique qui soit utile pour protéger la peau humaine vis à vis de la lumière bleue.

Ainsi, de manière surprenante, les Demandeurs ont développé un procédé d'extraction nouveau à partir des fèves de cacao permettant de mettre au point un extrait riche en peptides et osides qui s'est avéré démontrer une activité protectrice vis-à-vis de la lumière bleue en améliorant la fonction barrière de la peau ainsi qu'une activité préventive contre l'apparition du vieillissement cutané.

Le cacaoyer ou cacaotier ou encore appelé « Cacao » est un petit arbre à feuilles persistantes du genre Theobroma de la famille des Sterculiacées, selon la classification classique, ou des Malvacées, selon la classification phylogénétique. Le cacaoyer est une espèce tropicale originaire du Mexique domestiquée il y a environ 3 000 ans. C'est un arbre qui mesure de 10 à 15 mètres de haut, généralement taillé à 6 ou 8 mètres, cauliflore et à feuilles persistantes. Il fleurit à partir de 3 ans et donne fleurs, fruits et feuilles tout au long de l'année. L'arbre peut produire annuellement jusqu'à 100 000 fleurs de couleur blanche ou légèrement rosée. Elles apparaissent toute l'année sur des renflements du bois de l'arbre, appelés coussinets floraux. Par conséquent, on trouve au même moment des fleurs et des fruits sur l'arbre. Ses fruits, les « cabosses », sont de grosses baies allongées. Chaque cabosse peut peser jusqu'à 400 g pour 15 à 20 cm de long. Elles ont la particularité de grossir à la fois sur les branches maîtresses mais aussi directement sur le tronc de l'arbre. Leurs caractéristiques sont très variables d'une population à l'autre mais aussi au sein d'une même population. La maturation des fruits dure, selon les génotypes, de 5 à 7 mois. En moyenne un arbre donne environ 150 cabosses par an. Les cabosses contiennent de nombreuses graines (entre 25 et 75) regroupées en épis et appelées fèves de cacao riches en amidon, en matières grasses et en alcaloïdes.

Les fèves de cacao sont agglomérées au centre de la cabosse en une masse comportant cinq rangées correspondant aux cinq loges de l'ovule. Elles ont une forme variable, ovoïde-aplatie et mesurent environ 25 mm de long, 15 mm de large et 8 mm d'épaisseur. Fraîches, elles sont gluantes car entourées d'une pulpe blanche appelée mucilage. L'amande de la fève de cacao, est formée par un gros embryon qui comporte deux cotylédons repliés sur eux-mêmes. Elle est de couleur variable, allant du blanc au violet foncé selon les variétés. Elle est enveloppée d'un tégument de couleur rose ou rouge pâle. Il est connu que les fèves de cacao contiennent environ 50 % de matière grasse appelée beurre de cacao, 5 % d'eau, 7 % d'amidon, 4 % de cellulose, 2 % de théobromine, 20 % de protéines et 6 % de substances minérales.

On connaît que le cacao présente des effets stimulants, toniques, nutritifs et anti-stress et diverses substances ont été extraites du cacao en raison de telles propriétés. En cosmétique notamment, le beurre de cacao de Theobroma cacao est utilisé pour son action nourrissante sur la peau. Le beurre contient 53 % de matière grasse, (triglycérides comportant principalement les acides oléique, stéarique et palmitique) aux vertus super-nourrissantes, il a pour spécificité d'être riche en insaponifiables, composés de phytostérols et de squalène. Les premiers ont une action antioxydante, cicatrisante et apaisante (contre les brûlures et les gerçures). Le second, naturellement présent dans la peau et le sébum, aide à régénérer le ciment lipidique de la couche cornée. C'est pourquoi on retrouve le beurre de cacao comme ingrédient dans des beurres de massage pour la peau ainsi que dans des baumes hydratants, apaisants et protecteurs. Il est aussi apprécié dans les soins pour cheveux très secs et indisciplinés, ainsi que pour les pointes abîmées.

On connaît par ailleurs du document brevet FR2810242 une composition cosmétique et/ou dermatologique à base d'extrait protéique de cacao comprenant au moins des polyphénols, des acides aminés et une fraction insaponifiable. Lesdites compositions sont utiles pour le traitement et la prévention des signes du vieillissement cutané. Dans ce document, toute la fève de cacao est utilisée, et notamment le beurre de cacao, pour obtenir après hydrolyse enzymatique totale des protéines un mélange comprenant de 0,5 - 3 % ou 0,5 - 5 % d'insaponifiables, d'acides aminés et de polyphénols.

On connaît également du document brevet WO20080153341 une composition cosmétique comprenant un extrait de fève de cacao pour favoriser la pigmentation des poils et/ou des cheveux. Le procédé de préparation de l'extrait de fève comprend l'élimination des lipides, des acides nucléiques et des glucides de la fève. Le procédé d'obtention de l'extrait de fève est centré sur une hydrolyse enzymatique des protéines pour générer des polypeptides, des peptides et des acides aminés libres.

On connaît encore du document brevet US2013/0035291 un produit bioactif comprenant des peptides de 5 à 20 acides aminés et provenant d'un hydrolysat de cacao, ledit produit étant destiné à l'alimentation et pour traiter les maladies neurodégénératives. Le procédé mise en œuvre dans ce document permet d'obtenir des fractions spécifiques de peptides purifiés, exemptes de sucres.

Le procédé d'extraction selon la présente invention, mis au point par les Demandeurs sur les fèves de cacao, est nouveau par rapport aux documents cités ci-dessus. Ce procédé est optimisé pour sélectionner et garantir dans l'hydrolysat obtenu des fèves de cacao une forte teneur de molécules cibles d'intérêt comprenant des peptides et osides particuliers. L'hydrolysat peptidique et osidique selon l'invention ne comprend pas de molécules insaponifiables ni d'acides aminés libres et les traces de polyphénols présentes dans l'hydrolysat confirment un profil différent de celui du brevet WO200195872.

L'invention a donc pour objet un hydrolysat peptidique et osidique des fèves de Theobroma cacao L comprenant majoritairement des peptides et des osides obtenu par le procédé tel que défini dans la revendication 1.

De plus l'invention a pour second objet un procédé d'obtention selon l'invention, comprenant les étapes suivantes selon lesquelles :
a) on met en dispersion en phase aqueuse des fèves non fermentées, séchées et broyées de *Theobroma cacao* L. ;
b) on effectue un traitement enzymatique de la dispersion aqueuse obtenue à l'étape a) en deux temps, dans un premier traitement on utilise au moins une carbohydrase puis dans un second traitement on utilise au moins une protéase, la carbohydrase étant choisie parmi une pectinase, une cellulase, une arabanase, une hémicellulase, une xylanase et une béta-glucanase et la protéase étant de type alcalin, neutre ou acide, de préférence de type alcalin à activité endopeptidase ;
c) on dénature les enzymes par traitement thermique ;
d) on effectue la récupération de l'hydrolysat enzymatique par séparation solide / liquide ;
e) on purifie l'hydrolysat par ultrafiltration et nanofiltration, puis éventuellement ;
f) on effectue une lyophilisation de l'hydrolysat obtenu à l'étape e).

L'invention a pour objet une composition cosmétique, caractérisée en ce qu'elle comprend, à titre d'agent actif protecteur, une quantité efficace d'un hydrolysat de fèves de Theobroma cacao L selon l'invention, et un milieu physiologiquement acceptable.

Enfin, l'invention a pour objet une composition selon l'invention, pour son utilisation pour protéger la peau des effets néfastes de la lumière bleue, notamment du stress oxydatif généré par la lumière bleue, pour améliorer la fonction barrière de la peau et l'utilisation cosmétique pour lutter contre l'apparition des signes du vieillissement cutané et du photo-vieillissement.

L'invention et les avantages qui en découlent seront mieux compris à la lecture de la description et des modes de réalisation non limitatifs qui suivent, rédigée au regard des figures annexées dans lesquelles :

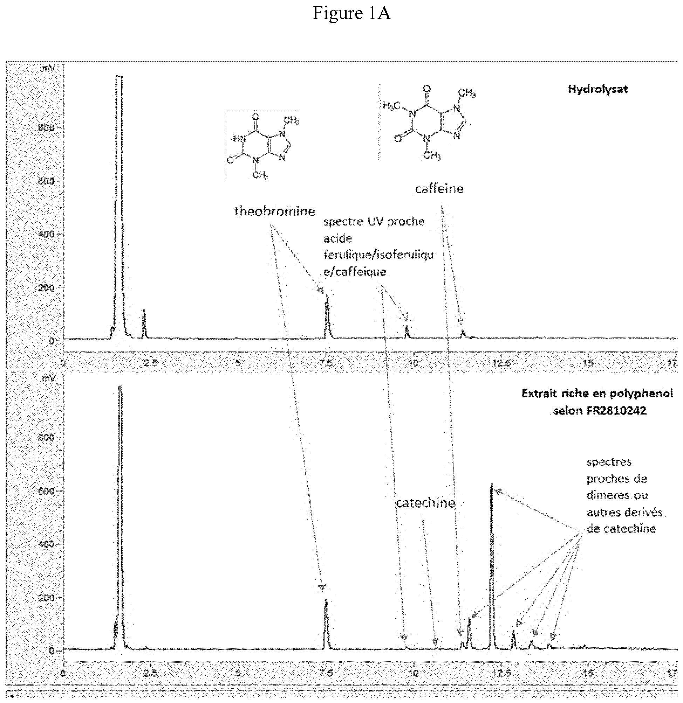

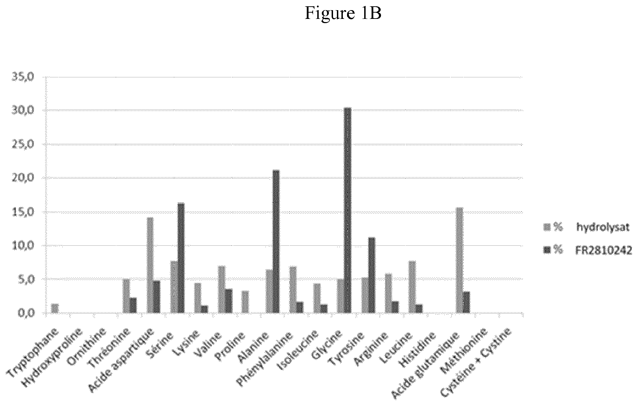

- La figure 1 représente la comparaison :
   - des profils polyphénoliques de l'hydrolysat selon l'invention et de l'extrait hydro alcoolique riche en polyphénols selon FR2810242 et
   - de l'aminogramme de l'hydrolysat selon l'invention, réalisé par hydrolyse totale des peptides par un acide fort tel que l'acide chlorhydrique 6 N pendant 24 à 48 heures, avec celui donné dans le document FR2810242.
- La figure 2 représente le niveau d'opsine-1, opsine-2 et opsine-3 après exposition à la lumière bleue (425 nm et 470 nm)
- La figure 3A représente la quantification de l'expression du collagène 1 par l'hydrolysat de cacao selon l'invention
- La figure 3B représente la quantification de l'expression de l'élastine par l'hydrolysat de cacao selon l'invention
- La figure 3C représente la quantification de l'expression de la fibrilline 1 par l'hydrolysat de cacao selon l'invention
- La figure 3D représente les résultats de l'hydrolysat de cacao selon l'invention sur la méthylation de l'ADN.

Dans cette description, à moins qu'il n'en soit spécifié autrement dans le texte, il est entendu que :
- lorsqu'un intervalle est donné, il inclut les bornes supérieure et inférieure dudit intervalle,
- les % sont exprimés en poids/poids dans le texte, sauf indication contraire.

Il est également indifféremment utilisé dans la description l'expression « hydrolysat peptidique et osidique de fève de *Theobroma cacao L* » ou « hydrolysat peptidique et osidique » ou « hydrolysat de cacao selon l'invention » ou « hydrolysat ».

Dans la présente invention, on entend par
✔ " quantité efficace " la quantité nécessaire de molécules actives pour obtenir le résultat recherché, à savoir, permettre notamment d'obtenir une protection cutanée vis à vis de la lumière bleue, sans que cette quantité ne soit toxique.
✔ "majoritairement des peptides et des osides" une quantité supérieure à 50%, préférentiellement supérieure à 60%, préférentiellement encore supérieure à 70% et pouvant atteindre environ 90% (poids/poids) de matière sèche en peptides et en oses, préférentiellement 90% du poids de la matière sèche.
✔ "hydrolysat peptidique et osidique" on entend un hydrolysat comprenant majoritairement ou essentiellement des peptides et des oses (mono et oligosaccharides). Les protéines et les polysaccharides naturellement présents dans les fèves ont été hydrolysés en peptides, en oligosaccharides et en monosaccharides, avantageusement l'hydrolyse est une hydrolyse enzymatique.
✔ «Application topique », le fait d'appliquer ou d'étaler le principe actif selon l'invention, ou une composition le contenant, à la surface de la peau, d'une muqueuse ou des phanères.
✔ « Cosmétiquement acceptable », que le principe actif selon l'invention, ou une composition le contenant, est approprié pour entrer en contact avec la peau ou une muqueuse sans provoquer de réactions de toxicité ou d'intolérance.
✔ « Physiologiquement acceptable » qui convient à une utilisation topique, en contact avec la peau humaine, ou à une utilisation par d'autres voies d'administration, par exemple la voie orale ou l'injection dans la peau, sans risque de toxicité, d'incompatibilité, d'instabilité, de réponse allergique.

L'invention a donc pour premier objet un hydrolysat peptidique et osidique des fèves de *Theobroma cacao L* comprenant majoritairement des peptides et des osides selon la revendication 1.

L'hydrolysat selon l'invention est obtenu à partir de fèves de *Theobroma cacao L,* comme matière de départ, qui peuvent comprendre indifféremment la fève seule ou la fève et son enveloppe, préférentiellement on utilisera les fèves comprenant la fève et son enveloppe.

Les fèves utilisées sont des fèves de *Theobroma cacao L* non fermentées et séchées.

Les fèves de *Theobroma cacao L* récoltées, lavées et séchées proviennent de préférence du pérou. Elles peuvent provenir d'un mélange de différentes variétés ou provenir d'au moins une des trois variétés Criollo, Forastero et Trinitario.

Dans un mode de réalisation préférentiel selon l'invention, les fèves proviendront de la variété Criollo et plus particulièrement de la sous variété Porcelana. De plus, les fèves de Cacao ne subiront pas les étapes essentielles du processus d'obtention du chocolat, c'est à dire la fermentation et la torréfaction car ces étapes ont un effet néfaste sur les protéines et les sucres pour cette application.

Selon l'invention, l'hydrolysat peptidique et osidique selon l'invention est obtenu par un traitement enzymatique réalisé avec au moins une carbohydrase et au moins une protéase. Le traitement enzymatique est effectué en deux temps, un premier traitement utilise les carbohydrases pour aboutir principalement à des di et monosaccharides ou mono-osides, puis un second traitement utilise des protéinases pour obtenir les peptides.

Selon l'invention, la carbohydrase est choisie parmi les pectinases, cellulases, arabanases, hémicellulases, xylanases et β-glucanases et la protéase est de type alcaline, neutre ou acide, de préférence de type alcaline à activité endopeptidase.

La carbohydrase sera avantageusement un cocktail de carbohydrases comprenant au moins une polygalacturonase, une pectine methyl esterase, une beta glucanase, une beta mannanase, une beta glucosidase, une cellulase, une hémicellulase, une arabanase et une xylanase.

L'hydrolysat de cacao non lyophilisé selon l'invention comprend de 20 à 70% de peptides et de 5 à 40% d'osides. L'hydrolysat de cacao lyophilisé sans support de séchage selon l'invention comprend plus de 90 pourcent de matière sèche comprenant de 20 à 70% de peptides et de 5 à 40% d'osides.

Dans un autre mode de réalisation très préféré selon l'invention, les peptides et osides présents dans l'hydrolysat selon l'invention présentent un poids moléculaire compris entre 200 Da et 10 kDa.

Avantageusement, l'hydrolysat selon l'invention ne contient pas d'insaponifiables.

Avantageusement encore, l'hydrolysat selon l'invention ne comprend pas d'acides aminés libres.

Enfin, l'hydrolysat selon l'invention comprend un maximum de 0,05% en polyphénols, lesquels sont majoritairement des molécules de type méthylxanthines, comme indiqué dans la figure 1.

Les fèves broyées de *Theobroma cacao* L utilisées dans l'étape a) du procédé selon l'invention sont préalablement récoltées, lavées, séchées puis délipidées par pressage pour aboutir à un tourteau de fève de cacao. Ce tourteau est utilisé pour la mise en œuvre de l'étape a) du procédé selon l'invention.

Par séchage, on entend la phase après récolte au cours de laquelle les fèves sont déshydratées afin de réduire suffisamment la teneur en eau des fèves pour garantir des conditions favorables de stockage ou de transformation ultérieure des fèves.

A titre d'exemples, les fèves peuvent être séchées naturellement par exposition à l'air libre (au soleil ou à l'ombre) pendant une durée d'au maximum 10 à 20 jours ou avantageusement délicatement séchées artificiellement en séchoir avec de l'air chaud soufflé, en contrôlant l'humidité relative, à une température comprise entre 40°C et 60°C.

Préférentiellement, après avoir été avantageusement lavées, les fèves sont séchées, puis broyées voire cryobroyées et délipidées.

Par fèves délipidées, on entend tous les procédés permettant d'éliminer tout ou partie des lipides des fèves de cacao tels que l'extraction solide/liquide par un solvant volatile, l'extraction par fluide supercritique, ou encore par pressage.

Dans l'étape a) du procédé selon l'invention, les fèves et notamment le tourteau de pressage est préférentiellement mis à 10% de matière sèche en solution dans l'eau.

Le traitement enzymatique selon l'étape b) du procédé selon l'invention est effectué avantageusement en deux temps, un premier traitement utilise les carbohydrases pour hydrolyser les parois des cellules végétales, puis un second traitement utilise des protéinases pour hydrolyser les protéines.

Dans un mode de réalisation préféré du procédé selon l'invention, la carbohydrase est choisie parmi les pectinases, cellulases, arabanases, hémicellulases, xylanases et β-glucanases et la protéase, de type alcaline, neutre ou acide, est de préférence de type alcaline avec une activité endopeptidase.

La carbohydrase sera avantageusement un cocktail de carbohydrases comprenant au moins une polygalacturonase, une pectine methyl esterase, une beta glucanase, une beta mannanase, une beta glucosidase, une cellulase, une hémi cellulase, une arabanase et une xylanase.

Les enzymes utilisées dans l'étape b) du procédé selon l'invention son avantageusement comprises entre 0,1% et 3,0% en poids par rapport au poids des fèves à traiter à traiter.

Dans un mode de réalisation préféré selon l'invention on utilisera plus de pectinases que de cellulases. Ainsi, on utilisera préférentiellement un ratio 2 : 1 de pectinases par rapport aux cellulases.

L'étape c) de dénaturation des enzymes est effectuée à une température comprise entre 80 et 90°C pendant une durée comprise entre 15 minutes à 2 heures, de préférence les conditions de traitement thermique sont de 85°C pendant 45 minutes.

Préférentiellement, lors des différentes étapes a) à d) du procédé selon l'invention, la température est comprise entre 20 et 90 °C et le pH est compris entre 3,0 et 11,0.

La récupération de l'hydrolysat enzymatique effectué selon l'étape c) du procédé selon l'invention est effectuée en réalisant une séparation solide/liquide par différents procédés connus de l'homme de l'art tels que la centrifugation, l'essorage, la filtration, de façon à obtenir un extrait exempt de particules solides. C'est avantageusement par centrifugation que la phase liquide est récupérée.

Cette étape de séparation solide-liquide est suivie d'une étape d) de purification par ultrafiltration, afin d'éliminer les protéines résiduelles et les enzymes dénaturées, avec une membrane ayant un seuil de coupure compris entre 10 et 15 kDa, de préférence de 10 kDa, et récupération du perméat. Une diafiltration est avantageusement effectuée, en diluant le retentât afin d'améliorer les performances séparatives et ainsi récupérer un maximum de composés d'intérêt.

On entend par « diafiltration » un rinçage du retentât par le même volume d'eau que ce retentât, de façon à faciliter le passage et ainsi récupérer le maximum de molécules dont le poids moléculaire est inférieur au seuil de filtration.

Cette étape d) peut aussi comprendre une nanofiltration afin d'éliminer éventuellement des acides aminés libres et des sels minéraux. Par exemple, le seuil de coupure sera compris entre 100 Da (Daltons) et 300 Da, avantageusement entre 130 et 300 Da, typiquement de 200 Da.

Dans un autre mode de réalisation, après la première étape d'ultrafiltration à 10 kDa, il est réalisé une seconde étape d'ultrafiltration sur membrane 5 kDa. Cette étape permet de récupérer la fraction du retentât contenant des peptides et osides dont le poids moléculaire est compris entre 5 kDa et 10 kDa.

Enfin, le perméat de cette ultrafiltration à 5 kDa est à son tour purifié par nanofiltration afin d'éliminer des acides aminés libres et des minéraux et obtenir un hydrolysat comprenant des peptides et osides compris entre 200 Da et 5 kDa.

L'hydrolysat ainsi obtenu peut ensuite être encore dilué dans de l'eau ou dans tout mélange de solvants contenant de l'eau. Ainsi, l'hydrolysat de cacao selon l'invention peut avantageusement être dilué dans un ou plusieurs solvants physiologiquement acceptables, tels que l'eau, le glycérol, l'éthanol, le propanediol, le butylène glycol, le dipropylène glycol, les diglycols éthoxylés ou propoxylés, les polyols cycliques ou tout mélange de ces solvants.

Un troisième objet selon l'invention concerne une composition cosmétique, caractérisée en ce qu'elle comprend, à titre d'agent actif protecteur, une quantité efficace d'un hydrolysat de fèves de *Theobroma cacao L* selon l'invention, et un milieu physiologiquement acceptable.

Dans un mode de réalisation préférentiel selon l'invention, l'hydrolysat de cacao est présent dans la composition à une concentration de 0,001% à 20% par rapport au poids total de la composition, préférentiellement de 0,1 à 10%, préférentiellement encore de 0,2 à 5%, plus préférentiellement encore de 0,5 à 1,5%. Pour préparer la composition, l'hydrolysat de cacao selon l'invention peut se trouver sous forme liquide ou lyophylisée.

Les compositions selon l'invention sont destinées plus particulièrement à une administration par voie topique. Ces compositions doivent donc contenir un milieu cosmétiquement acceptable, c'est-à-dire compatible avec la peau et les phanères, et couvrent toutes les formes cosmétiques. Ces compositions pourront notamment être sous forme de crèmes, émulsions huile-dans-eau, ou eau-dans-huile ou émulsions multiples, solutions, suspensions, gels, laits, lotions, sticks ou encore de poudres, et adaptées à une application sur la peau, les lèvres et/ou les phanères. Ces compositions comprennent les excipients nécessaires à leur formulation, tels que solvants, épaississants, diluants, tensioactifs, anti-oxydants, colorants, conservateurs, parfums. Elles peuvent être utilisées comme produit de soin et/ou comme produit de maquillage de la peau.

Les compositions selon l'invention comprennent, en outre, tout additif communément utilisé dans le domaine d'application envisagé ainsi que les adjuvants nécessaires à leur formulation, tels que des solvants, des épaississants, des diluants, des anti-oxydants, des colorants, des filtres solaires, des agents auto-bronzants, des pigments, des charges, des conservateurs, des parfums, des absorbeurs d'odeur, des actifs cosmétiques ou pharmaceutiques, des huiles essentielles, des vitamines, des acides gras essentiels, des tensioactifs, des polymères filmogènes, etc.

L'INCI Dictionary & Handbook ("International Nomenclature of Cosmetic Ingrédients 13ème Ed. 2010) publié par "the Personal Care Products Council, Inc.", Washington, D.C.) décrit une grande variété, sans limitation, d'ingrédients cosmétiques et pharmaceutiques habituellement utilisés dans l'industrie du soin de la peau, qui conviennent pour être utilisés comme ingrédients additionnels dans les compositions selon la présente invention.

Des exemples non limitatifs de ces classes d'ingrédients additionnels incluent : les agents cicatrisants, les agents anti-âge, les agents anti-rides, les agents anti-atrophie, les agents hydratants, les agents adoucissants, les agents antibactériens, les agents antiparasitaires, les agents antifongiques, les agents fongicides, les agents fongistatiques, les agents bactéricides, les agents bactériostatiques, les agents antimicrobiens, les agents anti-inflammatoires, les agents anti-prurigineux, les agents anesthésiques, les agents antiviraux, les agents kératolytiques, les agents anti-radicaux libres, les agents anti-séborrhéiques, les agents anti-pelliculaires, les agents modulant la différenciation, la prolifération ou la pigmentation cutanée, les agents accélérateurs de pénétration, les agents desquamants, les agents stimulant ou inhibant la synthèse de mélanine, les agents blanchissants, dépigmentants, ou éclaircissants, les agents propigmentants, les agents auto bronzants, les agents inhibant la NO-synthase, les agents antioxydants, les agents piégeurs de radicaux libres et/ou anti-pollution atmosphérique, les agents anti-glycation, les agents raffermissants, les agents stimulant la synthèse des macromolécules dermiques ou épidermiques et/ou les agents capables de prévenir ou inhiber leur dégradation, les agents stimulant la synthèse de collagène, les agents stimulant la synthèse d'élastine, les agents stimulant la synthèse de décorine, les agents stimulant la synthèse de laminine, les agents stimulant la synthèse de défensine, les agents stimulant la synthèse de chaperon, les agents stimulant la synthèse de d'aquaporine, les agents stimulant la synthèse d'acide hyaluronique, les agents stimulant la synthèse de lipides et de composants du stratum corneum (céramides, acides gras, ...), les agents inhibant la dégradation du collagène, les agents inhibant la dégradation de l'élastine, les agents stimulant la prolifération des fibroblastes, les agents stimulant la prolifération des kératinocytes, les agents stimulant la prolifération des adipocytes, les agents stimulant la prolifération des mélanocytes, les agents stimulant la différenciation des kératinocytes, les agents stimulant la différenciation des adipocytes, les agents inhibant l'acétylcholinestérase, les agents stimulant la synthèse des glycosaminoglycanes, les agents réparant l'ADN, les agents protégeant l'ADN, les agents anti-démangeaison, les agents pour le traitement et/ou le soin de la peau sensible, les agents raffermissants, les agents anti-vergetures, les agents astringents, les agents régulant la production du sébum, les agents dermo-relaxants, les agents adjuvants de guérison, les agents stimulant la réépithélialisation, les agents adjuvants de réépithélialisation, les facteurs de croissance de cytokine, les agents calmants, les agents anti-inflammatoires, les agents agissant sur la circulation et/ ou microcirculation capillaire, les agents stimulant l'angiogenèse, les agents inhibant la perméabilité vasculaire, les agents agissant sur le métabolisme cellulaire, les agents destinés à améliorer la jonction dermo-épidermique, les agents induisant la pousse des cheveux et/ou des poils, les agents inhibant ou ralentissant la pousse des cheveux et/ou des poils, les agents myorelaxants, les agents anti-pollution et/ou anti-radicalaires, les agents stimulant la lipolyse, les agents amincissants, les agents anti-cellulite, les agents agissant sur la microcirculation, les agents agissant sur le métabolisme des cellules, les agents nettoyants, les agents de coiffage du cheveu, les stimulants de la pousse des cheveux, les écrans solaires, les écrans totaux, les agents de maquillage, les détergents, les produits pharmaceutiques, les agents émulsifiants, les émollients, les solvants organiques, les agents antiseptiques, les actifs déodorants, les milieux physiologiquement acceptables, les surfactants, les agents abrasifs, les absorbants, les composants esthétiques comme les parfums, les pigments, les teintures, colorants et colorants naturels, les huiles essentielles, les agents de toucher, les astringents cosmétiques, les agents anti-acné, les agents anti-coagulation, les agents anti-mousse, les antioxydants, les liguants, les additifs biologiques, les enzymes, les inhibiteurs enzymatiques, les inducteurs enzymatiques, les coenzymes, les agents chélatants, les extraits végétaux et dérivés de plantes, les huiles essentielles, les extraits marins, les agents venant d'un procédé de biofermentation et/ou de biotechnologie, les sels minéraux, les extraits cellulaires, les écrans solaires (les agents photo-protecteurs organiques ou minéraux qui sont actifs contre les rayons ultraviolets A et/ou B) les céramides, les peptides, les tampons, les agents de volume, les agents chélatants, les additifs chimiques, les colorants, les biocides cosmétiques, les dénaturants, les astringents médicinaux, les analgésiques externes, les agents filmogènes, comme les polymères, pour exacerber les propriétés filmogènes et la substantivité de la composition, les dérivés quaternaires, les agents augmentant la substantivité, les agents opacifiants, les ajusteurs et régulateurs de pH (ex. triethanolamine), les propellants, les agents réducteurs, les séquestrants, les agents décolorants et/ou éclaircissants de la peau, les agents conditionnant de la peau (i.e., humectants, incluant miscellanées et occlusifs), les substances retenant l'humidité, les alpha hydroxy acides, les béta hydroxy acides, les hydratants, les enzymes hydrolytiques épidermiques, les agents apaisants et/ou cicatrisants, les agents traitant la peau, les agents anti-rides, les agents capables de réduire ou traiter les poches sous les yeux, les agents exfoliants, les épaississants, les adoucissants, les polymères gélifiants, les vitamines et leurs dérivés, les agents mouillants, les agents pelants, les agents calmants, les agents curatifs de la peau, les lignanes, les conservateurs (i.e.phenoxyethanol et parabens), les anti-UV, les agents cytotoxiques, anti néoplasiques, agents modifiant la viscosité, les solvants non volatils, des agents perlants, les agents antitranspirants, les dépilatoires, vaccins, eau parfumée, agent restructurant la peau, les excipients, les charges, les minerais, les agents anti-mycobactériens, les agents anti-allergéniques, les antihistaminiques H1 ou H2, les anti-irritants, les agents stimulant le système immunitaire, les agents inhibant le système immunitaire, les agents répulsifs d'insectes, les lubrifiants, les pigmentants ou colorants, les agents hypopigmentants, les agents photo-stabilisants, et leurs mélanges, tant qu'ils sont physiquement et chimiquement compatibles avec les autres ingrédients de la composition et spécialement avec les actifs de la présente invention.

Par ailleurs, la nature de ces ingrédients additionnels ne doit pas altérer de manière inacceptable les bénéfices des actifs de l'invention. Ces ingrédients additionnels peuvent être synthétiques ou naturels comme par exemple les extraits de plantes ou venir d'un procédé de biofermentation. Des exemples additionnels peuvent être trouvés dans l'INCI Dictionary & Handbook.

De tels ingrédients additionnels peuvent être choisis dans le groupe comprenant : les sucres aminés, glucosamine, D-glucosamine, N-acetyl- glucosamine, N-acetyl-D-glucosamine, mannosamine, N-acetyl mannosamine, galactosamine, N-acétyl galactosamine, vitamine B3 et ses dérivés, le niacinamide, déhydro-acétate de sodium, l'acide déhydroacétique et ses sels, phytosterols, composés d'acide salicylique, hexamidines, composés dihydroxyproline de dialkanoyl, extraits et dérivés de soja, equol, isoflavones, flavonoïdes, phytantriol, farnesol, géraniol, bisabolol, peptides et leurs dérivés, di -, tri -, tétra -, penta -, et hexapeptides et leurs dérivés, lys-thr-thr-lys-ser, palmitoyl-lys-thr-thr-lys-ser, carnosine, composés d'acide aminé N-acyl, rétinoïdes, propionate de rétinyl, rétinol, palmitate de rétinyl, acétate de rétinyl, rétinal, acide rétinoïque, vitamines hydrosolubles, ascorbates, vitamine C, glucoside ascorbyl, palmitate ascorbyl, magnesium ascorbyl phosphate, sodium ascorbyl phosphate, vitamines et leurs sels et dérivés, provitamines et leurs sels et dérivés, ethyl panthenol, vitamine B et ses dérivés, vitamine B1, vitamine B2, vitamine B6, vitamine B12, vitamine K et ses dérivés, acide pantothénique et ses dérivés, éther éthylique de pantothenyl, panthenol et ses dérivés, panthenol ethylique, dexpanthenol, biotine, acides aminés et leurs sels et les dérivés, acides aminés hydrosolubles, asparagine, alanine, indol, acide glutamique, vitamines insolubles dans l'eau, vitamine A, vitamine E, vitamine F , vitamine D et ses composés, mono-, di -, et triterpénoïdes, bêta-ionol, cedrol, et leurs dérivés, acides aminés insolubles dans l'eau, tyrosine, tryptamine, matériaux particulaires, hydroxytoluène butylé, hydroxyanisole butylé, allantoine, nicotinate de tocophérol, tocophérol, esters de tocophérol, palmitoyl-gly-his-lys, phytostérol, hydroxy acides, acide glycolique, acide lactique, acide lactobionique, kétoacides, acide pyruvique, acide phytique, acide lysophosphatidique, stilbenes, cinnamates, resveratrol, kinétine, zeatin, dimethylaminoethanol, peptides naturels, les peptides de soja, sels de sucres acides, gluconate de manganèse, gluconate de zinc, olamine de piroctone, 3,4,4'- trichlorocarbanilide, triclocarban, pyrithione de zinc, hydroquinone, acide kojique, acide ascorbique, magnésium ascorbyl phosphate, ascorbyl glucoside, pyridoxine, l'aloe vera, les alcools de terpène, allantoine, bisabolol, glycyrrhizinate dipotassique, acide de glycérol, de sorbitol, de pentaerythritol, de pyrrolidone et ses sels, dihydroxyacetone, erythrulose, glycéraldéhyde, tartaraldehyde, l'essence de clou de girofle, le menthol, le camphre, l'essence d'eucalyptus, eugénol, le lactate de menthyle, le distillat d'hamamélis, copolymère d'eicosene et vinyl pyrrolidone, iodopropyl butylcarbamate, un polysaccharide, un acide gras essentiel, un salicylate, un acide glycyrrhétinique, des caroténoïdes, des céramides et des pseudo-céramides, un lipide complexe, les huiles en générale d'origine naturelle telles le beurre de karité, l'huile d'abricot, l'huile d'onagre, l'huile de pruneau, l'huile de palme, l'huile de monoï, l'huile de kahai, hydroquinone, l'HEPES, la procystéine, l'O-octanoyl-6-D-maltose, le sel disodique d'acide méthyl glycine diacétique, les stéroïdes tels que la diosgénine et les dérivés de la DHEA, la DHEA déhydroépiandrostérone et/ou un précurseur ou dérivé chimique ou biologique, le N-éthylcarbonyl-4-para-aminophénol, les extraits de myrtille, les phytohormones, les extraits de levure *Saccharomyces cerevisiae,* les extraits d'algues, les extraits de soja, de lupin, de maïs et/ou de pois, l'alvérine et ses sels, en particulier le citrate d'alvérine, les extraits de petits houx et de marron d'inde et leurs combinaisons, un inhibiteur de métalloprotéinases, les extraits de *Schinus molle.*

Dans tous les cas, l'homme de métier veillera à ce que ces adjuvants ainsi que leurs proportions soient choisis de telle manière à ne pas nuire aux propriétés avantageuses recherchées de la composition selon l'invention.

Un quatrième objet selon l'invention concerne l'utilisation cosmétique d'une composition selon l'invention, pour protéger la peau des effets néfastes de la lumière bleue et améliorer la fonction barrière de la peau, ainsi que pour lutter contre l'apparition des signes du vieillissement et du photo-vieillissement cutané.

Par « signes du vieillissement cutané » on entend toute modification de l'aspect extérieur de la peau due au vieillissement chronologique comme les rides et ridules, les crevasses, les poches sous les yeux, les cernes, le flétrissement, la perte d'élasticité et/ou de tonus de la peau, le ternissement ou le manque d'éclat, les défauts pigmentaires mais également toute modification interne de la peau qui ne se traduit pas systématiquement par un aspect extérieur modifié comme, par exemple, l'amincissement et la perte de densité du derme, l'épaississement de la couche cornée.

Par « signes du photo-vieillissement cutané » on entend toute dégradation de la peau consécutive à une exposition aux rayonnements UV comme l'apparition prématurée de rides fines autour des yeux et de la bouche et de rides d'expression sur le front; la télangiectasie (petits vaisseaux sanguins dilatés) sur le nez, les joues et le cou, les taches pigmentaires diverses telles que les taches de rousseur et les lentigines solaires, les irrégularités du teint, la perte généralisée de tonus de la peau, la peau des lèvres qui devient plus tendue, perd de sa couleur et s'amincit, la peau peut aussi s'épaissir et devenir rugueuse. On entend également toute modification interne de la peau qui ne se traduit pas systématiquement par un aspect extérieur modifié comme, par exemple, l'épaississement des parois des vaisseaux, la modification de la forme des fibroblastes, le ralentissement de la synthèse du collagène et une désorganisation des fibrilles de collagène, une accumulation de matériel anormal et amorphe contenant de l'élastine (l'élastose solaire). A titre illustratif des exemples de mode de réalisation de l'invention sont décrits ci-dessous.

### Exemple 1 : Préparation d'un hydrolysat de cacao à partir des fèves de cacao

Les fèves utilisées comprennent leur enveloppe et sont d'origine péruvienne, de la variété Criollo et plus particulièrement de la sous-variété Porcelana.

On procède ainsi :
a) Mise en solution de tourteau de fèves de cacao délipidées par pressage, à 10% de matière sèche (MS) dans l'eau ;
b) Hydrolyse enzymatique des carbohydrates par action d'un cocktail de cellulases (0,5% / MS) et de pectinases (1,0%/ MS), comprenant une polygalacturonase, une pectine methyl esterase, une beta glucanase, une beta mannanase, une beta glucosidase, une cellulase, une hémicellulase, une arabanase et une xylanase, à un pH de 4,5 et une température de 55°C pendant 2 heures ;
c) Suivie d'une hydrolyse enzymatique par une protéase alcaline de type endopeptidase (0.5% MS), à un pH de 9,0 et une température de 55°C pendant 4 heures ;
d) Traitement thermique à 85°C pendant 45 minutes afin de dénaturer les enzymes ;
e) Centrifugation et récupération de la phase liquide ;
f) Ultrafiltration et diafiltration de la phase liquide sur membrane 10 kDa et récupération du perméat ;
g) Nanofiltration du perméat d'ultrafiltration sur membrane 200 Da et récupération du retentât.

L'hydrolysat de cacao obtenu dans le retentât à l'étape g) contient entre 3,0 à 4,0% de matière sèche.

Cette matière sèche comprend notamment 20 à 70% en poids de peptides et 5 à 40% d'osides présentant un poids moléculaire compris entre 200 Da et 10 kDa.

L'hydrolysat peut ensuite être dilué dans de l'eau ou dans tout mélange de solvants contenant de l'eau. Ainsi, l'hydrolysat de cacao selon l'invention peut avantageusement être dilué dans un ou plusieurs solvants physiologiquement acceptables, tels que l'eau, le glycérol, l'éthanol, le propanediol, le butylène glycol, le dipropylène glycol, les diglycols éthoxylés ou propoxylés, les polyols cycliques ou tout mélange de ces solvants.

### Exemple 2 : Préparation d'un hydrolysat de cacao à partir des fèves de cacao

Les étapes a) à g) sont identiques à celles effectuées dans l'exemple 1. Puis on procède ainsi :
h) Lyophilisation du retentât de nanofiltration avec 50% en poids de maltodextrine (support de séchage) par rapport à l'extrait sec de ce retentât.

L'hydrolysat peptidique de cacao obtenu dans le retentât de l'étape h) contient entre 94,0 à 98,0% de matière sèche.

Cette matière sèche comprend notamment 50% de maltodextrine, 10 à 35% en poids de peptides et 2 à 20% d'osides présentant un poids moléculaire compris entre 200 Da et 10 kDa.

### Exemple 3 : Préparation d'un hydrolysat de cacao à partir des fèves de cacao

Les étapes a) à g) sont identiques à celles effectuées dans l'exemple 2. Puis on procède ainsi :
h) Lyophilisation du retentât nanofiltré sans support de séchage.

L'hydrolysat peptidique de cacao obtenu à l'étape h) contient entre 94,0 à 98,0% de matière sèche.

Cette matière sèche comprend notamment 20 à 70% en poids de peptides et 5 à 40% d'osides présentant un poids moléculaire compris entre 200 Da et 10 kDa.

### Exemple 4 : Préparation d'un hydrolysat de cacao à partir des fèves de cacao

Les étapes a) à f) sont identiques à celles effectuées dans l'exemple 1 pour récupération de l'ultrafiltrat 1. Puis on procède ainsi :
g) Ultrafiltration du perméat ultrafiltré 1 sur membrane 5 kDa et récupération du retentât.
h) Lyophilisation du retentât ultrafiltré 2 sans support de séchage.

L'hydrolysat peptidique de cacao obtenu à l'étape h) contient entre 94,0 à 98,0% de matière sèche présentant un poids moléculaire compris entre 5 kDa et 10 kDa.

Cette matière sèche comprend notamment 20 à 70% en poids de peptides et 5 à 40% de sucres.

### Exemple 5 : Préparation d'un hydrolysat de cacao à partir des fèves de cacao

Les étapes a) à f) sont identiques à celles effectuées dans l'exemple 1 pour récupération du perméat de l'ultrafiltration 10 kDa (ultrafiltrat 1). On procède ensuite ainsi :
g) Ultrafiltration 2 de l'ultrafiltrat 1 sur membrane 5 kDa et récupération du perméat (ultrafiltrat 2) ;
h) Nanofiltration de l'ultrafiltrat 2 sur membrane 200 Da et récupération du retentât.

L'hydrolysat peptidique et osidique de cacao obtenu à l'étape h) contient entre 3,0 à 4,0% de matière sèche présentant un poids moléculaire compris entre 200 Da et 5 kDa.

Cette matière sèche comprend notamment 20 à 70% en poids de peptides et 5 à 40% de sucres.

### Exemple 6 : Comparaison de l'hydrolysat selon l'invention avec l'extrait obtenu selon FR2810242

Le brevet FR2810242, cité comme art antérieur, propose une composition cosmétique qui contient une combinaison de 0,05 à 5% en poids d'aminoacides extraits de cacao, 0,05 à 5% en poids de polyphénols extraits de cacao et 0,05 à 3% en poids de concentré d'insaponifiables.

En tant qu'extrait comparatif, il a été réalisé un extrait hydro-alcoolique riche en polyphénols (nommé extrait riche en polyphénol dans les exemples suivants) selon le document FR2810242 de la manière suivante :
Les polyphénols sont extraits par macération du tourteau délipidé à 10% d'extrait sec dans l'éthanol à 70% pendant 1 heure à 50°C, puis l'éthanol est évaporé sous vide.

L'extrait est ensuite dosé par la méthode de Folin Ciocalteu qui permet de doser l'ensemble des polyphénols. La méthode utilise l'acide gallique comme standard et les résultats sont exprimés en % massique équivalent acide gallique.

La comparaison quantitative des deux extraits est la suivante.

L'hydrolysat peptidique et osidique selon l'invention est caractérisé :
- par une teneur en polyphénols de 0,5% équivalent acide gallique. La composition qui contiendrait 10% de l'hydrolysat selon l'invention comprendrait au maximum 0,05% en polyphénols. Cela correspond aux teneurs minimales de FR2810242 compris entre 0,5 et 5% aux équivalences près (masse molaire de l'acide gallique en comparaison avec masse molaire des polyphénols présents dans l'hydrolysat).
- en qu'il ne contient pas ou peu d'acides aminés libres. Ils sont sous formes de peptides (hydrolyse partielle de protéines).
- en ce qu'il ne comprend pas de composés insaponifiables.

La comparaison qualitative des deux extraits

Il est important de noter que l'hydrolysat peptidique et osidique selon l'invention ne contient pas ou peu d'acides aminés libres. Ils sont sous formes de peptides (hydrolyse partielle de protéines). Ainsi, en termes de comparaison, il n'est pas justifiable de comparer la teneur en acide aminé et celle en peptide. Cependant il est intéressant de comparer les aminogrammes relatifs (profil relatif des acides aminés représentés dans les deux extraits).

Les profils polyphénoliques et en acides aminés de l'hydrolysat selon l'invention sont donnés dans la figure 1. Cette figure comprend :
- les profils polyphénoliques de l'hydrolysat selon l'invention et de l'extrait hydro alcoolique riche en polyphénols selon FR2810242 et
- les profils en acides aminés de l'hydrolysat selon l'invention hydrolysé complètement et les résultats de l'aminogramme donnés dans FR2810242.

L'extrait riche en polyphénols selon FR2810242 possède un profil polyphénolique complexe avec la présence potentielle de trois familles de molécules : des flavonoïdes (par exemple catéchine et dérivés), des phenyl-propanoides (par exemple acide férulique et dérivés) et des méthylxanthines (par exemple caféine et théobromine). En comparaison le profil polyphénolique de l'hydrolysat selon l'invention ne comporte plus les flavonoïdes mais toujours les methyl xanthines et phenyl-propanoides potentiels.

### Conclusion :

La comparaison qualitative de l'hydrolysat selon l'invention avec un extrait hydroalcoolique riche en polyphénols selon FR2810242 montre des différences significatives en termes de composition en polyphénols et en acides aminés (libres ou non).

### Exemple 7 : Evaluation des effets d'un hydrolysat peptidique et osidique de cacao selon l'exemple 1 sur le taux d'espèces oxygénées réactives cellulaires générées par la lumière bleue sur des kératinocytes en culture :

Le but de cette étude est d'évaluer l'effet d'un traitement par l'hydrolysat de cacao selon l'exemple 1, sur le niveau de stress oxydatif généré par une exposition à la lumière bleue, sur des kératinocytes humains normaux en culture. En parallèle, un traitement est effectué par un hydrolysat de cacao selon l'exemple 6 riche en polyphénols.

Une source de lumière bleue constituée par des LEDs est utilisée pour créer un stress à 415 nm et 470 nm. Les espèces oxygénées réactives (EORs) résultant de cette exposition sont déterminées au niveau cellulaire par une sonde fluorescente.

### Protocole :

Des kératinocytes humains normaux en culture sont préalablement traités par l'hydrolysat selon l'exemple 1 dilué à 0,1% volume/volume (soit au 1/1000^{eme}) dans un milieu de culture spécifique, deux fois par jour, tandis que des cultures contrôles sont maintenues en condition non-traitée. Après 24 heures, une partie des cultures contrôles et traitées est exposée à la lumière bleue (415 et 470 nm, à 3 mW/cm2 pendant 18 minutes), tandis que l'autre partie est maintenue à l'abri de la lumière. Après cette exposition, l'hydrolysat est appliqué deux fois par jour pendant à nouveau 24 heures. Puis, l'exposition à la lumière bleue est à nouveau répétée, suivi par le test de détection des EORs.

Pour ce faire, les cellules sont mises en présence d'une sonde fluorescente (CellROX® Green Reagent, Lifetechnologies) pendant 30 minutes à 37°C. Après fixation et rinçage, les cellules sont observées au microscope à Epi-fluorescence (Zeiss Axiovert 200M microscope). Une quantification de l'intensité de la fluorescence, proportionnelle à la quantité d'EORs présentes, est réalisée à l'aide du logiciel Volocity® image analysis software (PerkinElmer, Inc.) à partir des photographies obtenues.

Des traitements en présence d'hydrolysats de cacao obtenus selon les exemples 4 et 5 ont également été réalisés. Pour cela, les hydrolysats ont été préalablement dilués pour obtenir une concentration comparable à l'hydrolysat de l'exemple 1.

### Résultats :

Le traitement avec l'hydrolysat selon l'exemple 1 à 0,1% sur kératinocytes permet de réduire significativement le niveau d'EORs générées par une exposition à la lumière bleue (+90% d'intensité dans les cellules exposées à la lumière bleue contre +34% dans les cellules traitées et exposées, par rapport au contrôle non-traité). De plus, l'effet observé est supérieur à celui obtenu avec l'extrait selon l'exemple 6, riche en polyphénols, sur ces mêmes cellules.

Un traitement par les hydrolysats obtenus selon les exemples 4 et 5 ont également permis d'observer une réduction du niveau d'EORs générées par une exposition à la lumière bleue.

### Conclusion :

L'application de l'hydrolysat d'un hydrolysat de cacao obtenu selon l'un des exemples 1, 4 ou 5 permet de limiter le stress oxydatif généré par l'exposition à la lumière bleue, comme l'indique la diminution du taux d'EORs intracellulaires dans les kératinocytes traités avec l'hydrolysat.

### Exemple 8 : Evaluation des effets d'un hydrolysat de cacao selon l'exemple 1 sur le taux d'espèces oxygénées réactives mitochondriales générées par la lumière bleue sur des kératinocytes en culture :

Le but de cette étude est d'évaluer l'effet d'un traitement par un hydrolysat de cacao selon l'exemple 1, sur le niveau de stress oxydatif généré par une exposition à la lumière bleue, au niveau mitochondrial, sur des kératinocytes humains normaux en culture. En effet, la mitochondrie est une cible privilégiée des EORs et des dommages de l'ADN mitochondrial ont été observés suite à une exposition de cellules épithéliales de la rétine à la lumière visible¹³. En parallèle, un traitement est effectué par un hydrolysat de cacao selon l'exemple 6 riche en polyphénols sur ces mêmes cellules.

### Protocole :

Le protocole de traitement des cellules et d'exposition à la lumière bleue est identique à celui décrit dans l'exemple 7. Après ces étapes, les EORs mitochondriales sont révélées par une sonde fluorescente (MitoSOX™ Red Mitochondrial Superoxide Indicator, Invitrogen), mise au contact des cellules pendant 15 minutes à 37°C. Après rinçage et fixation, les cellules sont observées au microscope à Epi-fluorescence (Zeiss Axiovert 200M microscope). Une quantification de l'intensité de la fluorescence, proportionnelle à la quantité d'EORs présentes, est réalisée à l'aide du logiciel Volocity® image analysis software (PerkinElmer, Inc.) à partir des photographies obtenues.

Des traitements en présence d'hydrolysats de cacao obtenus selon les exemples 4 et 5 ont également été réalisés. Pour cela, les hydrolysats ont été préalablement dilués pour obtenir une concentration comparable à l'hydrolysat de l'exemple 1.

### Résultats :

L'exposition à la lumière bleue a entrainé une augmentation des EORs mitochondriales (+156% par rapport au contrôle non-traité). En présence de l'hydrolysat de cacao selon exemple 1 à 0,1%, on observe une forte baisse du taux d'EORs mitochondriales (+8% par rapport au contrôle non-traité). L'effet observé est supérieur à celui obtenu avec l'hydrolysat de cacao selon l'exemple 6 riche en polyphénols sur ces mêmes cellules.

Un traitement par les hydrolysats obtenus selon les exemples 4 et 5 ont également permis d'observer une réduction du niveau d'EORs générées par une exposition à la lumière bleue.

### Conclusion :

En complément des résultats rapportés dans l'exemple 7, ce test permet de conclure que l'application de l'hydrolysat de cacao selon exemple 1, 4, ou 5 permet de limiter le stress oxydatif généré par l'exposition à la lumière bleue, comme l'indique la diminution du taux d'EORs mitochondriales.

### Exemple 9 : Effet de l'hydrolysat de cacao selon exemple 1 sur la maintenance du niveau d'opsine-1, -2 et -3 après une exposition à la lumière bleue.

Le but de cette étude est d'observer l'effet d'une exposition à la lumière bleue telle que définie dans les exemples 7 et 8, sur le niveau de photo-récepteurs opsine-1, -2 et -3, sur des kératinocytes humains normaux traitée par l'hydrolysat de cacao selon exemple 1. Ces photo-récepteurs proviennent de l'expression des gènes OPN1-SW, OPN2 et OPN3 par les kératinocytes, et sont susceptibles d'induire des voies de signalisation en présence de la longueur d'onde de lumière bleue spécifique de chaque opsine¹².

### Protocole :

Des kératinocytes humains normaux en culture sont traités par l'hydrolysat selon l'exemple 1 dilué à 0,1% volume/volume (soit au 1/1000^{eme}), puis exposés à la lumière bleue selon le protocole décrit dans l'exemple 1. Trois heures après la seconde exposition, une détection des opsine-1, opsine-2 et opsine-3 est réalisée par une technique d'immuno-fluorescence indirecte.

Pour ce faire, les cellules sont rincées, fixées au paraformaldéhyde à 3,7% et perméabilisées au Triton à 0,1%, pendant 10 minutes. Après saturation des sites non spécifiques par de l'albumine de sérum bovin à 1% pendant 10 min, les cellules sont incubées avec une solution d'anticorps dirigés contre l'opsine-1 (OPSN Short), l'opsine-2 (rhodopsin) ou l'opsine-3 (panopsin) pendant une heure, puis d'une solution d'anticorps secondaire anti-lapin couplé à un fluorochrome (Alexa Fluor® 488, Invitrogen). Les cellules sont alors examinées au microscope à Epi-fluorescence (Zeiss Axiovert 200M microscope). Une quantification de la fluorescence, à l'aide du logiciel Volocity® image analysis software (PerkinElmer, Inc.) est réalisée à partir des photographies obtenues.

Des traitements en présence d'hydrolysats de cacao obtenus selon les exemples 4 et 5 ont également été réalisés. Pour cela, les hydrolysats ont été préalablement dilués pour obtenir une concentration comparable à l'hydrolysat de l'exemple 1.

### Résultats :

En présence de lumière bleue dans des conditions déterminées pour mimer une sur-exposition sur les kératinocytes en culture, l'intensité de marquage de l'opsine-1 est significativement diminuée par une exposition à 415 nm, correspondant à la longueur d'onde à laquelle cette opsine est sensible (-33% par rapport au contrôle). Dans la condition où les cellules ont été traitées par l'hydrolysat de cacao selon exemple 1 à 0.1%, le marquage de l'opsine-1 est préservée (-10% par rapport au contrôle). Dans les mêmes conditions, l'hydrolysat de cacao selon l'exemple 6 riche en polyphénols n'a pas permis d'observer un effet protecteur sur le niveau d'opsine-1 (-33% par rapport au contrôle non-traité).

Dans des conditions identiques de traitement, l'intensité de marquage de l'opsine-2 et de l'opsine-3 est diminuée lorsque les kératinocytes sont exposés à la lumière bleue (470 nm) (-43% et -27% respectivement, par rapport au contrôle non-traité). En présence de l'hydrolysat de cacao selon l'exemple 1 à 0,1%, le marquage des opsines apparait préservé (-28% et -10% respectivement, par rapport au contrôle non-traité). Dans les mêmes conditions, l'hydrolysat de cacao selon exemple 6 riche en polyphénols n'a pas permis d'observer un effet protecteur sur le niveau d'opsine-2 et d'opsine-3 (-42% et -30% respectivement, par rapport au contrôle non-traité).

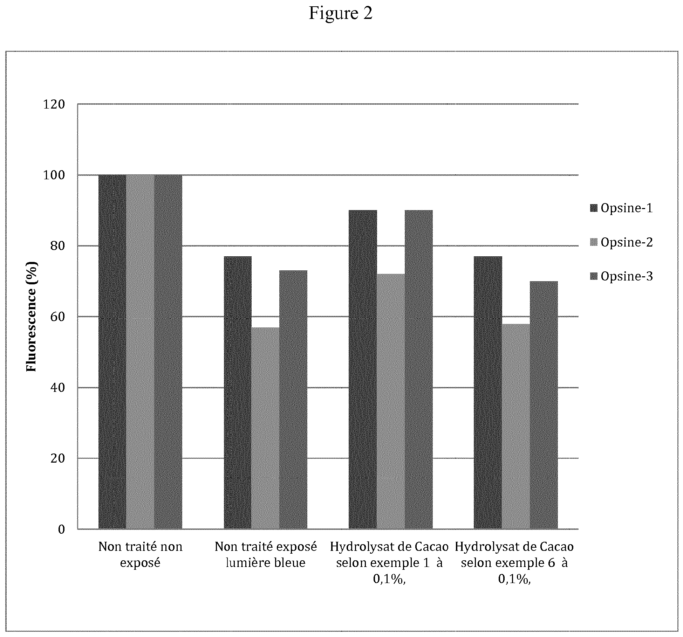

Les résultats sont présentés sur la figure 2.

Un traitement par les hydrolysats obtenus selon les exemples 4 et 5 ont également permis d'observer la maintenance du niveau d'opsine-1, -2 et -3 après une exposition à la lumière bleue.

### Conclusion :

L'hydrolysat de cacao obtenu selon l'un des exemples 1, 4 ou 5 a permis de maintenir le niveau d'opsine-1, -2 et -3 en présence d'un stress mimant une sur-exposition à la lumière bleue. Ces résultats démontrent que l'hydrolysat selon l'invention présente un effet protecteur vis-à-vis de la lumière bleue.

### Exemple 10 : Effet de l'hydrolysat de cacao selon exemple 1 sur le niveau d'expression des protéines circadiennes CRY-1 et PER-1, sur des kératinocytes exposés à la lumière bleue :

La présente étude a consisté à évaluer l'effet du traitement par l'hydrolysat de cacao selon exemple 1 sur la maintenance du niveau des protéines circadiennes CRY-1 et PER-1 en utilisant des kératinocytes soumis à une exposition à la lumière bleue, dans des conditions préalablement définies pour générer un stress sur ces cellules.

La modulation des gènes circadiens est une des conséquences de l'activation des photo-récepteurs au niveau des cellules de la rétine. L'expression des opsines dans les cellules rétiniennes est elle-même dépendante de l'expression de gène Clock, sous la régulation par des facteurs environnementaux tels que l'exposition à la lumière¹³.

### Protocole :

Des kératinocytes humains normaux en culture sont traités par l'hydrolysat de cacao selon l'exemple 1 dilué à 0,1% volume/volume (soit au 1/1000^{eme}) puis exposés à la lumière bleue selon le protocole décrit dans l'exemple 7. Des cultures traitées dans les mêmes conditions sont maintenues à l'obscurité. Six heures après la seconde exposition, le niveau d'expression des protéines circadiennes CRY-1 et PER-1 est révélé par la technique d'immunocytochimie selon un protocole standard tel que décrit dans l'exemple 9.

Après rinçage, fixation et saturation des sites non-spécifiques, les cellules sont incubées avec des anticorps primaires dirigés contre les protéines CRY-1 et PER-1, puis avec des anticorps secondaires couplés à un fluorochrome (Alexa Fluor® 488, Invitrogen). Les cellules sont ensuite examinées au microscope à Epi-fluorescence (Zeiss Axiovert 200M microscope). Une quantification de la fluorescence, à l'aide du logiciel Volocity® image analysis software (PerkinElmer, Inc.) est réalisée à partir des photographies obtenues.

Des traitements en présence d'hydrolysats de cacao obtenus selon les exemples 4 et 5 ont également été réalisés. Pour cela, les hydrolysats ont été préalablement dilués pour obtenir une concentration comparable à l'hydrolysat de l'exemple 1.

### Résultats :

Dans la condition où les cellules ont été maintenues dans le noir, l'hydrolysat de cacao selon exemple 1 à 0,1% a permis d'augmenter de +19% et +17% le niveau d'expression des protéines CRY-1 et PER-1, respectivement, par rapport au contrôle non traité. L'exposition à la lumière bleue (415 nm) entraîne une diminution significative de CRY-1 (-25% par rapport aux cellules non exposées), qui est contrecarré par le traitement avec l'hydrolysat de cacao selon exemple 1 (+20% par rapport au contrôle non exposé et non traité). Concernant le niveau d'expression de PER-1, il est également diminué dans les cellules exposées à la lumière bleue (415 nm) (-11% par rapport aux cellules non exposées) et apparait maintenu au-dessus du contrôle dans la condition exposée et traitée par l'hydrolysat (+27% par rapport au contrôle non exposé et non traité), dans ces mêmes conditions.

Un traitement par les hydrolysats obtenus selon les exemples 4 et 5 a également permis d'observer les mêmes résultats.

### Conclusion :

Le traitement par l'hydrolysat de cacao obtenu selon l'un des exemples 1, 4 ou 5 a permis de maintenir le niveau des protéines circadiennes CRY-1 et PER-1 dans les kératinocytes exposés à la lumière bleue. Ces résultats démontrent que l'hydrolysat selon l'invention présente un effet protecteur vis-à-vis de la lumière bleue.

### Exemple 11 : Effet de l'hydrolysat de cacao selon exemple 1 sur la maintenance du réseau de fibrilline-1 sur des biopsies de peau ex vivo exposées à un stress UVB ou à la lumière bleue :

Le but de cette étude est d'évaluer un effet protecteur potentiel de l'hydrolysat de cacao selon exemple 1 sur le réseau de fibrilline-1 en présence d'un stress généré par des UVB ou par une exposition à la lumière bleue.

Le réseau de fibres élastiques apparait comme une cible particulière du photo-vieillissement, notamment au niveau des changements de structure des micro fibrilles riches en fibrilline-1. Cette protéine est un constituant des fibres oxytalanes présentes dans le derme superficiel. Avec le photo-vieillissement, les microfibrilles d'élastine perdent leur forme caractéristique en candélabre en dessous de la jonction dermo-épidermique¹⁴.

### Protocole :

Des biopsies de peau humaine maintenues en culture sont traitées par l'hydrolysat de cacao selon l'exemple 1 dilué à 1% volume/volume (soit au 1/100^{eme}) dans du PBS, 2 fois par jour pendant 24 heures, puis irradiées par des UVB (100 mJ/cm2) ou exposées à la lumière bleue (470 nm, 3 mW/cm2 pendant 26 min). Des biopsies sont conservées en parallèle à l'obscurité. Le traitement est appliqué à nouveau 2 fois par jour pendant 24 heures, puis les biopsies subissent une seconde exposition aux UVB ou à la lumière bleue. Après à nouveau 24 heures de traitement, les biopsies sont recueillies afin de procéder à la détection de la fibrilline-1 par immunohistochimie.

Cette technique est réalisée à partir de coupes congelées, incubées en présence de l'anticorps anti-fibrilline-1 (monoclonal de souris). Après 1 heure d'incubation suivie de rinçages, les coupes sont incubées en présence de l'anticorps secondaire anti-souris couplé à un fluorophore (Alexa Fluor® 488, Invitrogen). Les coupes sont ensuite examinées au microscope à Epi-fluorescence (Zeiss Axiovert 200M microscope). Le réseau de microfibrilles d'élastine présent en dessous de la jonction dermo-épidermique est alors observé.

Des traitements en présence d'hydrolysats de cacao obtenus selon les exemples 4 et 5 ont également été réalisés. Pour cela, les hydrolysats ont été préalablement dilués pour obtenir une concentration comparable à l'hydrolysat de l'exemple 1.

### Résultats :

Pour les biopsies restées à l'obscurité, les microfibrilles marquées par la fibrilline-1 apparaissent plus longues en présence de l'hydrolysat de cacao selon exemple 1 à 1%, comparé aux biopsies non-traitées.

Dans la condition où les biopsies ont été exposées aux UVB, le réseau de microfibrilles apparait nettement diminué et il n'est plus possible de distinguer une orientation perpendiculaire des fibres. Dans la condition où les biopsies ont été traitées par l'hydrolysat de cacao selon exemple 1 à 1% et exposées aux UVB, on observe des fibres plus longues et perpendiculaires à la jonction dermo-épidermique, comparé aux biopsies exposées non traitées. Ces effets ont été également observés dans le cas d'une exposition à la lumière bleue.

Un traitement par les hydrolysats obtenus selon les exemples 4 et 5 a également permis d'observer les mêmes résultats.

### Conclusion :

Ces résultats indiquent un effet protecteur de l'hydrolysat de cacao obtenu selon l'un des exemples 1, 4 ou 5, vis-à-vis de la fibrilline-1, dans le cas d'un stress UVB et d'une exposition à la lumière bleue, suggérant un intérêt dans la prévention du photo-vieillissement.

### Exemple 12 : Effet de l'hydrolysat de cacao selon exemple 1 sur le niveau d'expression de svndecan-4 dans des biopsies de peau ex vivo :

Le but de la présente étude est d'évaluer l'effet potentiel de l'hydrolysat de cacao selon exemple 1 sur le taux de syndécan-4 sur des biopsies de peau humaine.

Le syndécan-4 est un protéoglycane à héparane-sulfate présent à la surface cellulaire où il joue le rôle de médiateur des interactions cellulaires, régulant les mécanismes d'adhésion cellulaire, de migration, de prolifération, d'endocytose et de méchano-transduction¹⁵.

### Protocole :

Les biopsies de peau humaine ont été traitées par l'hydrolysat de cacao selon l'exemple 1 dilué à 1% volume/volume (soit au 1/100^{eme}) dans du PBS, 2 fois par jour, pendant un total de 72 heures.

Puis le syndécan-4 a été révélé sur des coupes de ces biopsies par immunohistochimie. Dans ce but, les coupes sont mises au contact de l'anticorps primaire dirigé contre le syndécan-4 (polyclonal de lapin). Après incubation et rinçages, un anticorps secondaire anti-lapin couplé à un fluorophore (Alexa Fluor® 488, Invitrogen) est appliqué sur les coupes. Les coupes sont ensuite examinées au microscope à Epi-fluorescence (Zeiss Axiovert 200M microscope). Une quantification de la fluorescence, à l'aide du logiciel Volocity® image analysis software (PerkinElmer, Inc.) est réalisée à partir des photographies obtenues.

### Résultats :

L'hydrolysat de cacao selon exemple 1 a permis d'augmenter le taux de syndécan-4 de +18% par rapport aux biopsies contrôles non-traitées. Cet effet est observé dans une moindre mesure avec l'hydrolysat de cacao préparé selon exemple 6 riche en polyphénols (+12%).

### Conclusion :

L'hydrolysat de cacao selon exemple 1 a une activité sur le syndécan-4 dans des biopsies de peau, suggérant un effet potentiel sur les interactions entre les kératinocytes et leur environnement extra-cellulaire.

### Exemple 13 : Tests in vitro démontrant l'activité anti-âge et rajeunissante de l'hydrolysat de cacao selon l'exemple 2

Le derme fournit à l'épiderme un support solide, c'est également son élément nourricier. Il est principalement constitué de fibroblastes et d'une matrice extracellulaire composée majoritairement de collagènes, d'élastine et d'une substance, dite substance fondamentale. Ces composants sont synthétisés par les fibroblastes. La cohésion entre l'épiderme et le derme est assurée par la jonction dermo-épidermique.

Les collagènes sont les protéines majoritaires des matrices extracellulaires de la peau. A ce jour, 20 types de collagènes sont identifiés et notés de I à XX. Les collagènes majoritairement présents dans l'ensemble du derme sont les collagènes de type I et III qui forment la matrice extracellulaire de l'ensemble du derme (ce sont ces collagènes qui constituent 70-80 % du poids sec du derme). Avec le vieillissement, le derme s'aminçit et des rides apparaissent à la surface de la peau. En conséquence, compte tenu du rôle important du collagène au niveau de l'intégrité de la peau et de sa résistance aux agressions externes de type mécaniques, la stimulation de la synthèse de ces collagènes, et notamment du collagène de type I, apparaît comme un moyen efficace pour pallier les signes du vieillissement cutané (revue par Tzaphlidou M., Micron 35 (2004) 173-177).

### 1 - Etude sur cellules jeunes versus cellules vieillies

L'étude qui suit a permis d'étudier l'effet de l'hydrolysat de cacao selon l'exemple 2 (nommé hydrolysat dans les figures) sur l'expression du collagène I, de l'Elastine et de la Fibrilline 1, constituants essentiels de la matrice extracellulaire du derme, afin d'évaluer son activité anti-vieillissante et anti-photo-vieillissante ainsi que rajeunissante. Cette dernière activité a également été évaluée en étudiant l'effet de l'hydrolysat de cacao selon l'exemple 2 sur la méthylation de l'ADN, cible de l'épigénétique.

Au cours du vieillissement les fibres de collagène et les fibres élastiques sont altérées du fait d'une synthèse réduite et d'une dégradation accrue, ayant pour conséquence une qualité de peau amoindrie, moins tonique et en perte d'élasticité.

Pour permettre une revendication de Rajeunissement cellulaire nous avons quantifié l'expression des protéines impliquées dans la structure de ces fibres au sein de cellules vieillies de manière intrinsèque (senescence réplicative) dans le cas du Collagène I et de l'Elastine, ou extrinsèque par irradiations UV répétées (photo-vieillissement) dans le cas de la Fibrilline 1.

Afin de comprendre le mécanisme de réactivation de l'expression des protéines observée, nous avons procédé à une analyse de la méthylation de l'ADN sur cellules vieillies de manière intrinsèque. En effet l'une des signatures de la sénescence cellulaire est l'accumulation de zones méthylées au sein de l'ADN, au niveau des Cytosines contenues dans les dinucléotides CpG. Ceci entraine l'inactivation de promoteurs et la diminution de l'expression de certains gènes.

### • Evaluation préalable de la cytotoxicité

La cytotoxicité des molécules a été évaluée sur les NHDF.

La cytotoxicité a été réalisée sur la concentration maximale testée soluble en solution aqueuse à 0,1% DMSO ainsi qu'à 7 dilutions de ½ log en ½ log.

Le composé a été mis en contact avec les cellules pendant 24 heures. Durant les 3 dernières heures du test calorimétrique prêt à l'emploi WST1 de Roche® a été introduit dans le milieu.

Ce réactif contient des sels de tetrazolium, un indicateur violet. Ce réactif est clivé en formazan, un indicateur jaune, par les cellules métaboliquement actives. Le niveau de coloration jaune est donc proportionnel au nombre de cellules vivantes. La mesure de l'absorbance se fait à 450nm.

Le test considère qu'une valeur inférieure à 90% du témoin indique une cytotoxicité du produit éventuelle (symbolisée par une ligne verte dans les graphes). Cela peut aussi indiquer que l'on diminue l'activité métabolique des cellules. Une valeur inférieure à 75% indique une cytotoxicité significative (symbolisée par une ligne rouge dans les graphes)

Par ailleurs les cellules ont été observées au microscope pour observer et comparer leur physionomie.

L'hydrolysat de cacao selon l'exemple 2 présente une forte cytotoxicité à 1000 ppm et une cytotoxicité plus faible mais significative entre 200 et 20ppm. L'hydrolysat de cacao selon l'exemple 2 ne présente pas de cytotoxicité significative entre 2 ppm et 20 ppm.

### Concentrations retenues pour être testées :

L'hydrolysat de cacao selon l'exemple 2 est testé aux concentrations suivantes 20 - 10 - 5 ppm.

### - Cibles Collagène I

Le Collagène I est le Collagène majoritaire qui apporte à la peau sa résistance mécanique.

Cette protéine représente 90 % du collagène d'un vertébré. Il constitue la trame de l'os (à comparer aux armatures du béton armé), et plus généralement des tissus conjonctifs banals. Il se trouve dans les os, la peau, les tendons, la cornée et les organes internes.

### • Méthode d'évaluation de l'effet de l'hydrolysat de cacao selon l'exemple 2 sur l'expression du Collagène I

Les NHDF (Normal Human Dermal Fibroblasts) jeunes ou vieillis par sénescence réplicative ont été ensemencés en plaque 96 puits et incubées 24h à 37°C, 5%CO2.

Les cellules ont été traitées 24h en présence des produits d'essai.

Les cellules ont ensuite été fixées avec de la formaline et l'expression des protéines a été détectée par immunofluorescence.

Les marquages fluorescents ont été imagés et quantifiés par microscopie automatisée (Arrayscan CellomicsTM). La fluorescence a été quantifiée par la bioapplication Compartimentai Analysis.

### • Evaluation de l'effet de l'hydrolysat de cacao selon l'exemple 2 sur l'expression du Collagène I

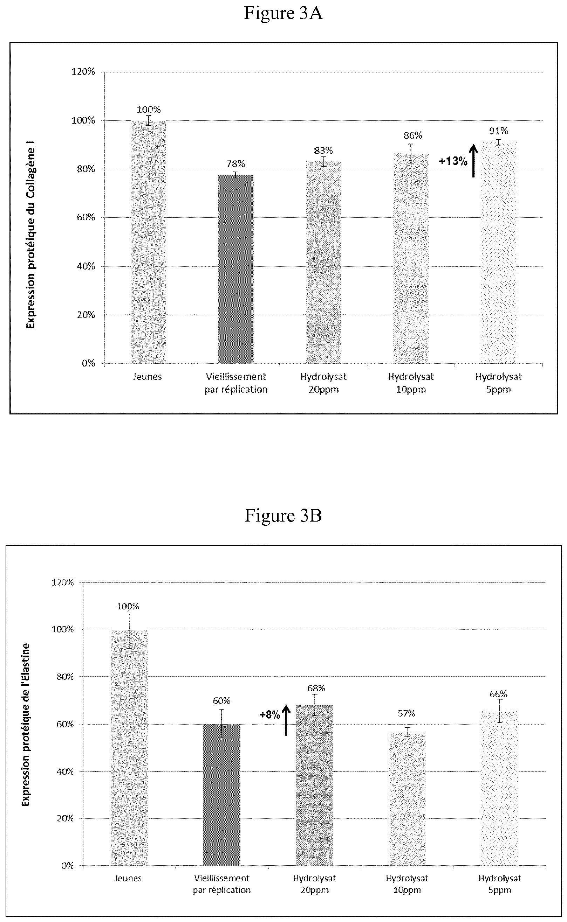

Les résultats sont donnés dans la figure 3A.

L'hydrolysat de cacao selon l'exemple 2 permet de réactiver l'expression de Collagène I par rapport aux cellules vieillies par réplication. En effet, les pourcentages d'expression protéique de la protéine ciblée obtenus aux trois concentrations testées d'hydrolysat de cacao selon l'exemple 2, sont supérieurs à celui obtenus avec le modèle de cellules âgées.

### Conclusion :

L'hydrolysat de cacao selon l'exemple 2, est un ingrédient qui agit sur le collagène I, cible du vieillissement cutané, en relançant l'expression protéique par rapport à des cellules vieillies non traitées et en la rétablissant partiellement en comparaison aux témoins jeunes.

### - Cible Elastine

L'élastine est une glycoprotéine structurale (comme la laminine et la fibronectine) entrant dans la composition de la MEC. L'élastine est une protéine de la famille des protéines fibreuses de type structural. Sécrétée par les fibroblastes essentiellement durant la période de croissance, elle possède des propriétés élastiques. Sa synthèse diminue avec l'âge et l'élastine se trouve remplacée par du collagène inextensible. Les vergetures sont un exemple visible de ce processus, qui est lié à des contraintes mécaniques. Le vieillissement cutané en est un deuxième exemple.

Dans la matrice extracellulaire l'élastine est synthétisée et sécrétée dans l'espace extracellulaire par les fibroblastes d'abord en proélastine, puis en tropoélastine. L'élastine est la composante majeure (jusqu'à 90 %) des fibres élastiques auxquelles s'ajoute la fibrilline. Donc, le collagène, associé à l'élastine et la fibrilline qui forment les fibres élastiques, par des liaisons transversales covalentes, sont les principaux constituants de la matrice extracellulaire. La production totale d'élastine s'arrête autour de la puberté. Après quoi, la quantité d'élastine disponible diminuera avec le temps.La dégradation de l'élastine est liée à l'action de l'élastase, une enzyme sécrétée par les fibroblastes. L'action enzymatique de l'élastase est inhibée par l'al-antitrypsine. L'inhibition de la dégradation crée un équilibre augmentant la stabilité de l'élastine.

Des traits distinctifs caractérisent l'élastine : l'élastine permet aux cellules de se lier et permet aux tissus biologiques de se former. Ainsi, le bon fonctionnement de la peau, des poumons, des vaisseaux sanguins, des tissus conjonctifs, de certains tendons et cartilages est étroitement lié aux caractéristiques de l'élastine. Comme son nom l'indique, l'élastine est élastique. À diamètre égal, elle est 5 fois plus élastique qu'un élastique. Elle peut s'étirer jusqu'à 150% de sa longueur au repos avant de se briser. Ainsi, elle permet aux tissus de s'étirer et de retrouver leur état initial après l'étirement, ce qui leur donne de la souplesse.

L'élastine se retrouve dans le derme de la peau, celui-ci agissant en tant que soutien. Au cours du vieillissement, par exemple, la perte d'élasticité et de tonicité du derme qui ne peut plus s'opposer aux effets de contraction des muscles sous-jacents donne lieu à l'apparition des rides. Par ailleurs, l'exposition aux ultraviolets augmente la dégradation de l'élastine.

### • Méthode d'évaluation de l'effet de l'hydrolysat de cacao selon l'exemple 2 sur l'expression de l'Elastine

Les NHDF (Normal Human Dermal Fibroblasts) jeunes ou vieillis par sénescence réplicative ont été ensemencés en plaque 96 puits et incubées 24h à 37°C, 5%CO2.

Les cellules ont été traitées 24h en présence des produits d'essai.

Les cellules ont ensuite été fixées avec de la formaline et l'expression des protéines a été détectée par immunofluorescence.

Les marquages fluorescents ont été imagés et quantifiés par microscopie automatisée (Arrayscan CellomicsTM). La fluorescence a été quantifiée par la bioapplication Compartimentai Analysis.

### • Evaluation de l'effet de l'hydrolysat de cacao selon l'exemple 2 sur l'expression de l'Elastine

Les résultats sont donnés dans la figure 3B.

L'hydrolysat de cacao selon l'exemple 2 permet de réactiver légèrement l'expression de l'Elastine par rapport aux cellules vieillies.

### • Conclusion

L'hydrolysat de cacao selon l'exemple 2, est un ingrédient qui agit sur l'Elastine, cible du vieillissement cutané, en relançant légèrement l'expression protéique par rapport à des cellules vieillies non traitées aux trois concentrations testées et en la rétablissant partiellement par rapport aux témoins jeunes.

### - Cible Fibrilline 1

La Fibrilline 1 est une protéine qui constitue les microfibrilles, lesquelles sont associées aux fibres élastiques et participent à leur assemblage.

### • Méthode d'évaluation de l'effet de l'hydrolysat peptidique de Cacao sur l'expression de la Fibrilline 1

Les NHDF (Normal Human Dermal Fibroblasts) jeunes ou vieillis extrinsèquement par irradiations ont été ensemencés en plaque 96 puits et incubées 24h à 37°C, 5%CO2.

Les cellules destinées au vieillissement extrinsèque ont été irradiées 3 fois aux UVA avec 24h entre chaque irradiation ou aux 2 fois aux UVB avec 48h en chaque irradiation.

Les cellules ont été traitées 24h en présence des produits d'essai.

Les cellules ont ensuite été fixées avec de la formaline et l'expression des protéines a été détectée par immunofluorescence.

Les marquages fluorescents ont été imagés et quantifiés par microscopie automatisée (Arrayscan CellomicsTM). La fluorescence a été quantifiée par la bioapplication Compartimentai Analysis.

### • Evaluation de l'effet de l'hydrolysat de cacao selon l'exemple 2 sur l'expression de la Fibrilline 1

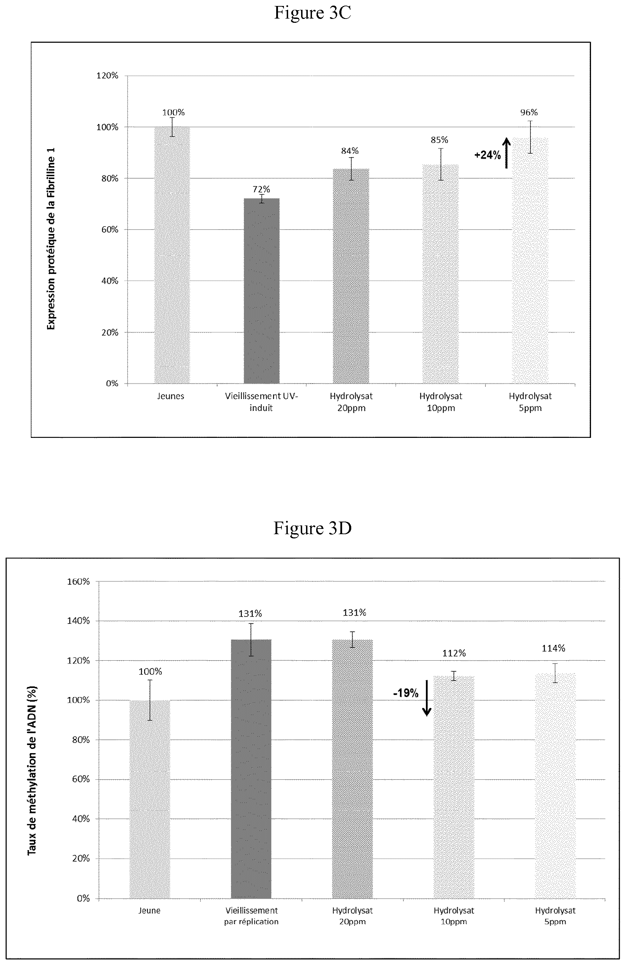

Les résultats sont donnés dans la figure 3C.

L'hydrolysat de cacao selon l'exemple 2 permet de réactiver l'expression de la Fibrilline 1 par rapport aux cellules vieillies.

### • Conclusion

L'hydrolysat de cacao selon l'exemple 2, est un ingrédient qui agit sur la Fibrilline 1, cible du vieillissement cutané, en relançant son expression protéique aux trois concentrations testées par rapport à des cellules vieillies non traitées et en la rétablissant presque totalement en comparaison aux témoins jeunes.

### - Cible Méthylation de l'ADN

La méthylation est une modification des extrémités N-terminales des histones. Elle peut s'effectuer soit sur des lysines soit sur des arginines et peut se concrétiser par l'ajout d'un, de deux ou de trois groupements méthyles. Selon les résidus méthylés et le nombre de groupements ajoutés, elle est associée à une activation ou une répression de la transcription. Longtemps considérée comme statique, la méthylation des histones s'avère être une modification réversible impliquée dans un processus dynamique, bien que plus stable que l'acétylation et la phosphorylation. Un nombre croissant d'histones déméthylases sont identifiés. De manière générale, ce type de modifications est antagoniste à l'acétylation, et la désacétylation des lysines doit précéder leur méthylation. Cet antagonisme entraîne la mise en place d'un certain équilibre dynamique entre les territoires hétérochromatiniens (généralement non exprimables et méthylés sur certains acides aminés clés) et euchromatiniens (généralement exprimables et acétylés). Par exemple, la Lysine 9 de l'histone H3 est connue pour être associée à une répression de la chromatine environnante lorsqu'elle est méthylée. Cette méthylation est reconnue par une protéine, HP1, qui se fixe donc sur H3 méthylée. À son tour, HP1 attire la protéine Suv39, une Histone MethylTransférase, qui pourra méthyler la lysine 9 de l'histone H3 du nucléosome voisin, et ainsi de suite. On voit donc, comment, de proche en proche, les histones H3 seront méthylées et la chromatine sera condensée. Cependant, cette invasion hétérochromatinienne sera stoppée si la lysine 9 de H3 rencontrée est déjà acétylée. Ainsi se met en place un équilibre compétitif entre domaines chromatiniens exprimés et réprimés. Les modifications des queues d'histones jouent le rôle de « marques » épigénétiques qui entraînent le recrutement de différentes classes de protéines, puisque les lysines acétylées ou méthylées sont reconnues par des domaines protéiques différents. De plus, le recrutement de certains facteurs au niveau de la chromatine nécessite l'existence préalable de modifications d'histones et de protéines déjà liées. Le code des histones est donc interprété dans le contexte d'autres facteurs associés à la chromatine et c'est la combinaison d'interaction entre les histones modifiées et d'autres facteurs qui détermine si une protéine est recrutée à la chromatine. Tous les tissus des organismes sont affectés par le vieillissement. Ce processus est lié aux modifications épigénétiques telles que les changements de méthylation au niveau de résidus cytosine spécifiques de l'ADN, tel que décrit dans de nombreuses publications. Le rôle des modifications épigénétiques sur le vieillissement, l'accumulation de divisions cellulaires et de macromolécules détériorées contribuent à un phénotype âgé. Les événements environnementaux et aléatoires peuvent de plus modifier ce phénotype par l'intermédiaire de mécanismes épigénétiques tels que la méthylation de l'ADN et la méthylation et l'acétylation des histones. La potentielle réversibilité des modifications épigénétiques fait d'eux des cibles attractives pour le traitement des pathologies liées au vieillissement.

### • Méthode d'évaluation de l'effet de l'hydrolysat de cacao selon l'exemple 2 sur la méthylation de l'ADN

Les NHDF (Normal Human Dermal Fibroblasts) jeunes ou vieillis par sénescence réplicative ont été ensemencés en plaque 96 puits et incubées 24h à 37°C, 5%CO2.

Les cellules ont été traitées 24h en présence des produits d'essai.

Les cellules ont ensuite été décollées en présence de trypsine et lysées. L'ADN génomique a été précipité à l'Ethanol.

Le taux de méthylation de l'ADN a été dosé par ELISA en utilisant le kit Enzo : 5-Methylcytosine DNA ELISA kit. Les valeurs sont représentées à partir de 50% pour une meilleure visibilité des résultats.

### • Evaluation de l'effet de l'hydrolysat de cacao selon l'exemple 2 sur la méthylation de l'ADN

Les résultats sont donnés dans la figure 3D.

L'hydrolysat de cacao selon l'exemple 2 permet de baisser le taux de méthylation dans les NHDF vieillis par réplication à 10 et 5 ppm pour se rapprocher de celui observé dans les cellules jeunes.

### • Conclusion

L'hydrolysat de cacao selon l'exemple 2 , est un ingrédient qui agit sur la diminution du taux de méthylation de l'ADN, cible de l'épivieillissement, et possède donc la potentialité d'une activité rajeunissante.

### - Conclusion générale sur l'activité anti-âge et rajeunissante

Au cours du vieillissement les fibres de collagène et les fibres élastiques sont altérées du fait d'une synthèse réduite et d'une dégradation accrue. Pour démontrer que l'hydrolysat de cacao selon l'exemple 2 agit sur le rajeunissement cellulaire, l'expression des protéines impliquées dans la structure de ces fibres au sein de cellules vieillies de manière intrinsèque (senescence réplicative) dans le cas du Collagène I et de l'Elastine, ou extrinsèque par irradiations UV répétées (photo-vieillissement) dans le cas de la Fibrilline 1 ont été quantifiées.

L'hydrolysat de cacao selon l'exemple 2 permet de relancer l'expression des 3 marqueurs.

Dans le cadre de l'analyse de la méthylation de l'ADN l'hydrolysat de cacao selon l'exemple 2 présente des résultats homogènes dans cette expérience, il diminue la méthylation à 2 concentrations testées sur 3. Cet effet donne une information sur le mode d'action de ce produit permettant de réactiver l'expression de protéines de la matrice extracellulaire, lui conférant son effet rajeunissant.

L'hydrolysat de cacao selon l'exemple 2 est par conséquent un ingrédient ou principe actif présentant une activité biologique anti-âge, antivieillissement et rajeunissant. Il agit de manière cohérente sur 3 cibles du vieillissement cutané en relançant l'expression protéique par rapport à celle observée dans les cellules vieillies. Il présente également une activité novatrice sur l'épigénétique comme le démontre son action sur la méthylation de l'ADN au sein de cellules matures. Enfin, il démontre une action anti-âge en profondeur par son efficacité sur la synthèse des protéines de la matrice extracellulaire.

### Exemple 14 : Crème de soin

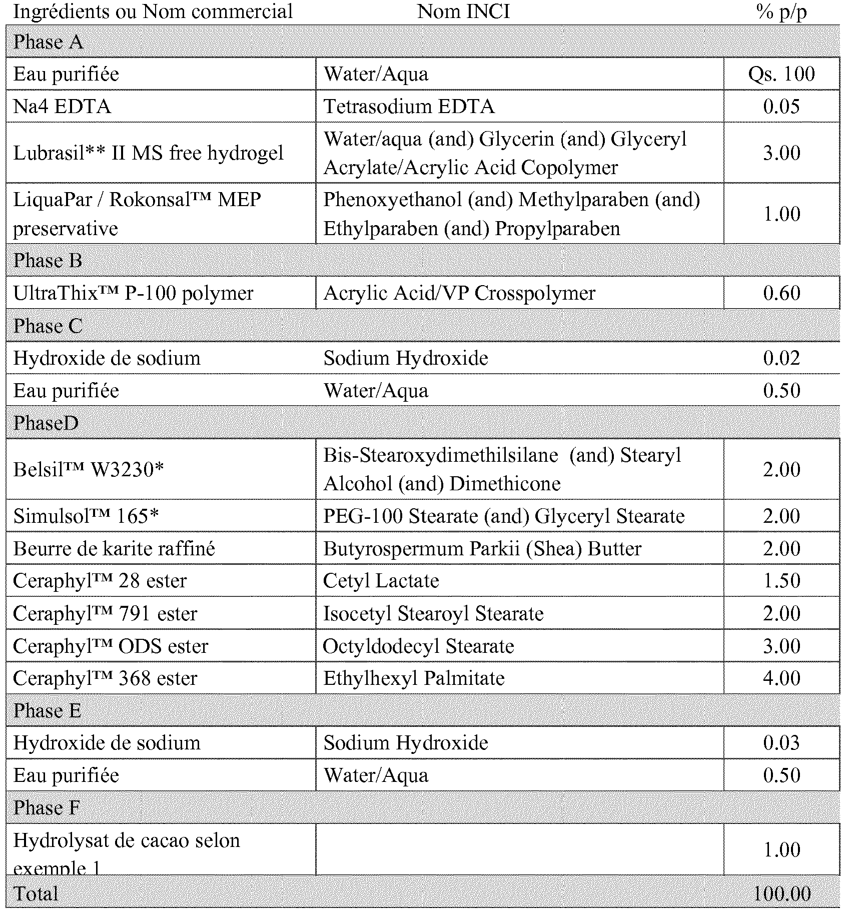

### Procédure

1. Verser la phase A dans le récipient principal et commencer à homogénéiser. Chauffer à 70-75°C.
2. Faire tomber en pluie dans l'UltraThix P-100 et mélanger bien pendant environ 30min.
3. Ajouter les ingrédients de la phase D dans un deuxième bécher et chauffer à 70-75°C.
4. Ajouter la phase C pré-mélangée dans la phase A jusqu'à homogénéisation complète
5. A 70-75°C ajouter la phase D dans le récipient principal et mélanger bien. L'émulsion doit être parfaitement homogène.
6. Commencer le refroidissement.
7. Quand le mélange atteint ∼50°C, ajouter la phase E pré-mélanger et mélanger bien.
8. A température ambiante, ajouter la phase F et mélanger jusquà obtenir un mélange uniforme.
9. Arrêter à 25°C

**Propriétés :**

| | |
|---|---|
| Apparence: | Crème blanche |
| pH: | 5,2 - 5,6 |
| Viscosité (D0) | 25000 - 50000 (Brookfield RVT/Spindle B/5 RPM/ 1 minute/25°C) |

La conservation de cette formule a été validée par un double test d'efficacité sur 28 jours. Toutefois, les conservateurs n'ont pas été optimisés à leur plus bas niveau d'efficacité.

### Exemple 15 : Fluide protecteurs pour activités d'extérieur (protection solaire SPF 30 in vitro)

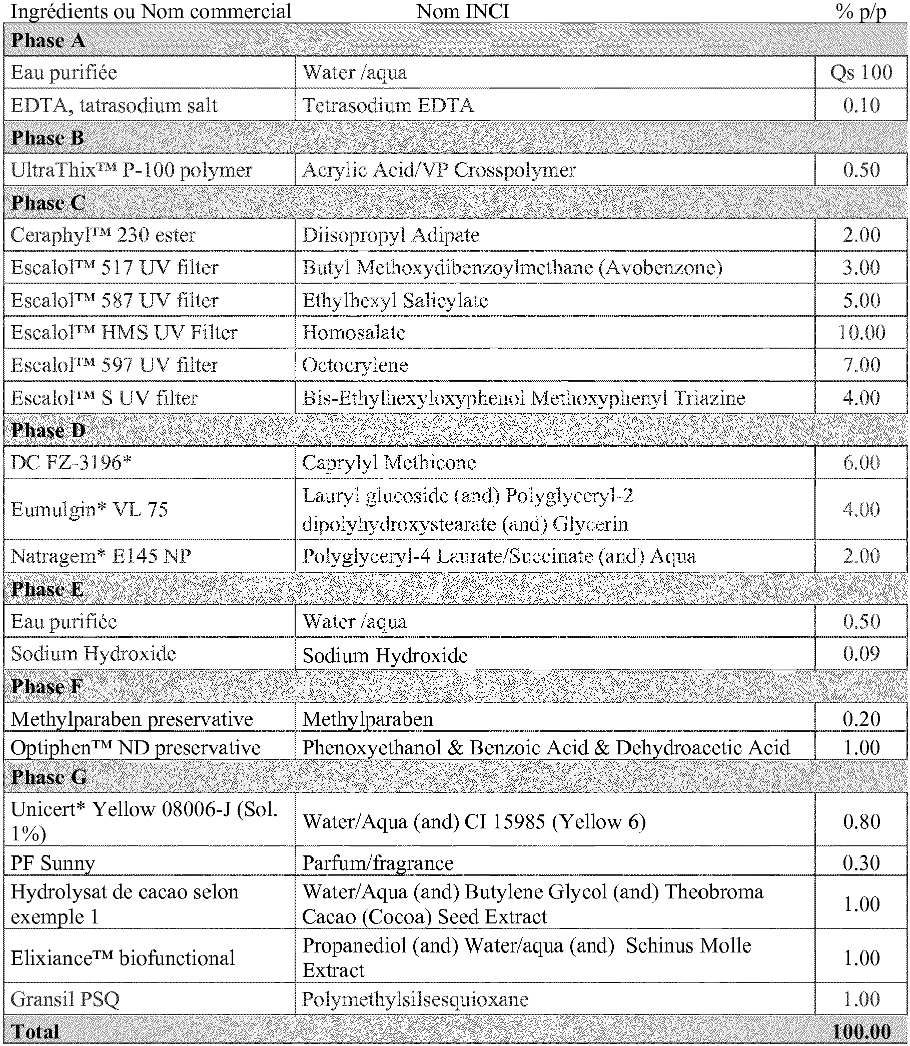

### Procédure

1. Ajouter l'eau dans le récipient principal et ajouter les ingrédients de la phase A à température ambiante
2. Faire tomber en pluie dans la phase A et mélanger jusqu'à homogénéité
3. Ajouter les ingrédients de la phase C dans un récipient annexe et chauffer à 65-70°C en mélangeant puis refroidir à température ambiante.
4. A température ambiante homogénéiser laphase C + la phase D
5. Ajouter CD dans le récipient principal et mélanger bien, jusqu'à obtenir une émulsion homogène
6. Prémélanger la phase E et ajouter dans le récipient principal tout en mélangeant
7. Prémélanger la phase F à 50°C ajouter dans le récipient principal tout en mélangeant
8. Ajouter les ingrédients de la phase G un à un et mixer bien jusqu'à homogénéisation
9. Stop à 25°C.

**Propriétés :**

| | |
|---|---|
| Apparence: | Fluide orange |
| pH: | 5,5 - 6,0 |
| Viscosité (D0) | Brookfield RVT/Spindle 3/5 RPM/ 1 minute/25°C: 5000 - 11000 cps |

La conservation de cette formule a été validée par un double test d'efficacité sur 28 jours. Toutefois, les conservateurs n'ont pas été optimisés à leur plus bas niveau d'efficacité.

### Références bibliographiques

1. Denda M, Fuziwara S. Visible radiation affects epidermal permeability barrier recovery: selective effects of red and blue light. J Invest Dermatol. 2008 May;128(5):1335-6).
2. Godley BF, Shamsi FA, Liang FQ, Jarrett SG, Davies S, Boulton M. Blue light induces mitochondrial DNA damage and free radical production in epithelial cells. J Biol Chem. 2005 Jun 3;280(22):21061-6
3. Seko Y, Pang J, Tokoro T, Ichinose S, Mochizuki M. Blue light-induced apoptosis in cultured retinal pigment epithelium cells of the rat. Graefes Arch Clin Exp Ophthalmol. 2001 Jan;239(1):47-52
4. Katz ML. Potential role of retinal pigment epithelial lipofuscin accumulation in age-related macular degeneration. Arch Gerontol Geriatr. 2002 May-Jun;34(3):359-70
5. Kuse Y et al. Damage of photoreceptor-derived cells in culture induced by light emitting diode-derived blue light, Sci Rep. 2014 Jun 9;4:5223
6. Kuse Y et al. Damage of photoreceptor-derived cells in culture induced by light emitting diode-derived blue light, Sci Rep. 2014 Jun 9;4:5223
7. Kuse Y et al. Damage of photoreceptor-derived cells in culture induced by light emitting diode-derived blue light, Sci Rep. 2014 Jun 9;4:5223
8. Fisher MR et al, Blue light irradiation suppresses dendritic cells activation in vitro. Exp Dermatol. 2013 Aug;22(8):558-60
9. Gold MH et al, Clinical Efficacy of Self-applied Blue Light Therapy for Mild-to-Moderate Facial Acne. J Clin Aesthet Dermatol. 2009 Mar;2(3):44-50
10. Haltaufderhyde K et al, Opsin expression in human epidermal skin. Photochem Photobiol. 2015 Jan-Feb;91(1):117-23
11. Poletini MO, Ramos BC, Moraes MN, Castrucci AM. Nonvisual Opsins and the Regulation of Peripheral Clocks by Light and Hormones. Photochem Photobiol. 2015 Sep-Oct;91(5):1046-55
12. Haltaufderhyde K et al, Opsin expression in human epidermal skin. Photochem Photobiol. 2015 Jan-Feb;91(1):117-23
13. Li P, Chaurasia SS, Gao Y, Carr AL, Iuvone PM, Li L. CLOCK is required for maintaining the circadian rhythms of Opsin mRNA expression in photoreceptor cells. J Biol Chem. 2008 Nov 14;283(46):31673-8
14. Langton AK, Sherratt MJ, Griffiths CE, Watson RE. A new wrinkle on old skin: the role of elastic fibres in skin ageing. Int J Cosmet Sci. 2010 Oct;32(5):330-9
15. Elfenbein A, Simons M. Syndecan-4 signaling at a glance. J Cell Sci. 2013 Sep 1;126(Pt 17):3799-804). Il a notamment comme ligand des facteurs de croissance tels que le fibroblast growth factor (FGF), vascular endothelial growth factor (VEGF) et platelet-derived growth factor (PDGF)

## Revendications

1. Hydrolysat peptidique et osidique de fèves de *Theobroma cacao L.,* **caractérisé en ce que** les peptides et osides présentent un poids moléculaire compris entre 200 Da et 10 kDa et **en ce que** ledit hydrolysat est obtenu par le procédé comprenant les étapes suivantes :
a) on met en dispersion en phase aqueuse des fèves non fermentées, séchées et broyées de *Theobroma cacao L. ;*
b) on effectue un traitement enzymatique de la dispersion aqueuse obtenue à l'étape a) en deux temps, dans un premier traitement on utilise au moins une carbohydrase puis dans un second traitement on utilise au moins une protéase, la carbohydrase étant choisie parmi une pectinase, une cellulase, une arabanase, une hémicellulase, une xylanase et une β-glucanase et la protéase étant de type alcalin, neutre ou acide, de préférence de type alcalin à activité endopeptidase ;
c) on dénature les enzymes par traitement thermique ;
d) on effectue la récupération de l'hydrolysat enzymatique par séparation solide / liquide ;
e) on purifie l'hydrolysat par ultrafiltration et nanofiltration, puis éventuellement ;
f) on effectue une lyophilisation de l'hydrolysat obtenu à l'étape e).

2. Hydrolysat peptidique et osidique de fèves de Theobroma cacao L. selon la revendication 1, **caractérisé en ce que** les fèves de l'étapes a) sont en outre délipidées.

3. Hydrolysat peptidique et osidique de fèves de *Theobroma cacao L.* selon l'une des revendications 1 ou 2, **caractérisé en ce que** les carbohydrases de l'étape b) sont un cocktail de carbohydrases comprenant au moins une polygalacturonase, une pectine methyl esterase, une beta glucanase, une beta mannanase, une beta glucosidase, une cellulase, une hémi cellulase, une arabanase et une xylanase.

4. Hydrolysat peptidique et osidique de fèves de *Theobroma cacao L.* selon l'une des revendications 1 à 3, **caractérisé en ce que** la concentration totale d'enzyme utilisée dans l'étape b) du procédé est comprise entre 0,1% et 3,0% en poids par rapport au poids de fèves à traiter.

5. Hydrolysat peptidique et osidique de fèves de *Theobroma cacao L.* selon l'une des revendications 3 ou 4, **caractérisé en ce que** les carbohydrases de l'étape b) comprennent 0,5% en poids du poids total de matière sèche de cellulases et 1% en poids du poids total de matière sèche de pectinase.

6. Hydrolysat peptidique et osidique de fèves de *Theobroma cacao L.* selon l'une des revendications 1 à 5, **caractérisé en ce que** les étapes a) à d) du procédé sont réalisés à une température comprise entre 20 et 90 °C et un pH compris entre 3,0 et 11,0.

7. Hydrolysat peptidique et osidique de fèves de *Theobroma cacao L.* selon l'une des revendications 1 à 6, **caractérisé en ce que** la séparation solide/liquide de l'étape d) est réalisée par différents procédés tels que centrifugation, essorage, filtration.

8. Hydrolysat peptidique et osidique de fèves de *Theobroma cacao L.* selon l'une des revendications 1 à 7, **caractérisé en ce que** à l'étape e) l'ultrafiltration est effectuée avec un seuil de coupure compris entre 5 et 15 kDa et la nanofiltration est effectuée avec un seuil de coupure compris entre 100 Da et 300 Da.

9. Composition cosmétique, **caractérisée en ce qu'**elle comprend, à titre d'agent actif, une quantité efficace d'un hydrolysat de fèves de *Theobroma cacao L.* selon l'une des revendications 1 à 8, et un milieu physiologiquement acceptable.

10. Composition selon la revendication 9, **caractérisée en ce que** l'hydrolysat de fèves de *Theobroma cacao L.* est présent dans la composition à une concentration de 0,001 à 20% par rapport au poids total de la composition, préférentiellement de 0,1 à 10%, préférentiellement de 0,2 à 5%, plus préférentiellement encore de 0,5 à 1,5%.

11. Composition selon l'une des revendications 9 ou 10, **caractérisée en ce qu'**elle est formulée pour être appliquée topiquement sur la peau.

12. Composition selon l'une des revendications 9 à 11 pour son utilisation pour protéger la peau des effets néfastes de la lumière bleue.

13. Composition pour son utilisation selon la revendication 12 pour protéger la peau du stress oxydatif généré par l'exposition à la lumière bleue.

14. Utilisation cosmétique d'une composition selon l'une des revendications 9 à 11 pour lutter contre l'apparition des signes du vieillissement et du photo-vieillissement cutané.

## Patentansprüche

1. Peptid- und Osidhydrolysat von *Theobroma cacao L.* Bohnen, **dadurch gekennzeichnet, dass** die Peptide und Oside ein Molekulargewicht zwischen 200 Da und 10 kDa haben und das Hydrolysat durch das Verfahren erhalten wird, das die folgenden Schritte umfasst:
a) nicht fermentierte, getrocknete und gemahlene *Theobroma cacao L.* Bohnen werden in einer wässrigen Phase dispergiert;
b) eine enzymatische Behandlung der in Schritt a) erhaltenen wässrigen Dispersion wird in zwei Stufen durchgeführt, wobei in einer ersten Behandlung mindestens eine Carbohydrase verwendet wird, dann in einer zweiten Behandlung mindestens eine Protease verwendet wird, wobei die Carbohydrase aus einer Pektinase, einer Cellulase, einer Arabanase, einer Hemicellulase, einer Xylanase und einer β-Glucanase ausgewählt wird und die Protease vom alkalischen, neutralen oder sauren Typ ausgewählt wird, vorzugsweise vom Typ alkalisch mit Endopeptinaseaktivität;
c) die Enzyme werden durch thermische Behandlung denaturiert;
d) die Gewinnung des enzymatischen Hydrolysats wird durch Fest/Flüssigtrennung erfolgt;
e) das Hydrolysat wird dann durch Ultrafiltration und Nanofiltation rückgewonnen, und gegebenfalls;
f) eine Lyophilisation des unter e) gewonnenen Hydrolysats wird durchgeführt.

2. Peptid- und Osidhydrolysat von Theobroma cacao L. Bohnen nach Anspruch 1, **dadurch gekennzeichnet, dass** die Bohnen von Schritt a) zusätzlich delipidiert werden.

3. Peptid- und Osidhydrolysat von *Theobroma cacao L.* Bohnen nach Anspruch 1 oder 2, **dadurch gekennzeichnet, dass** die Carbohydrasen von Schritt b) ein Cocktail aus Carbohydrasen ist, umfassend mindestens eine Polygalacturonase, eine Pektinmethylesterase, eine Beta-Glucanase, eine Beta-Mannanase, eine Beta-Glucosidase, eine Cellulase, eine Hemicellulase, eine Arabanase und eine Xylanase.

4. Peptid- und Osidhydrolysat von *Theobroma cacao L.* Bohnen nach Anspruch 1 bis 3, **dadurch gekennzeichnet, dass** die Gesamtkonzentration des in Schritt b) des Verfahrens verwendeten Enzyms zwischen 0,1 Gew.-% und 3,0 Gew.-% bezogen auf das Gewicht der zu behandelnden Bohnen liegt.

5. Peptid- und Osidhydrolysat von *Theobroma cacao L.* Bohnen nach einem der Ansprüche 3 oder 4, **dadurch gekennzeichnet, dass** die Carbohydrasen von Schritt b) 0,5 Gew.-% des Gesamtgewichts der Trockenmasse der Cellulasen und 1 Gew.-% des Gesamtgewichts der Trockenmasse der Pektinase umfassen.

6. Peptid- und Osidhydrolysat von *Theobroma cacao L.* Bohnen nach einem der Ansprüche 1 bis 5, **dadurch gekennzeichnet, dass** die Schritte a) bis d) des Verfahrens bei einer Temperatur zwischen 20 und 90 °C und einem pH-Wert zwischen 3,0 und 11,0 durchgeführt werden.

7. Peptid- und Osidhydrolysat von *Theobroma cacao L.* Bohnen nach einem der Ansprüche 1 bis 6, **dadurch gekennzeichnet, dass** die Fest-/Flüssigtrennung von Schritt d) durch verschiedene Vorgänge wie Zentrifugation, Schleudern, Filtration durchgeführt wird.

8. Peptid- und Osidhydrolysat von *Theobroma cacao L.* Bohnen nach einem der Ansprüche 1 bis 7, **dadurch gekennzeichnet, dass** bei Schritt e) die Ultrafiltration bei einer Trenngrenze zwischen 5 und 15 kDa und die Nanofiltration bei einer Trenngrenze zwischen 100 Da und 300 Da durchgeführt wird.

9. Kosmetische Zusammensetzung, **dadurch gekennzeichnet, dass** sie als Wirkstoff eine wirksame Menge eines Hydrolysats von *Theobroma cacao L.* Bohnen nach einem der Ansprüche 1 bis 8 und ein physiologisch verträgliches Medium umfasst.

10. Kosmetische Zusammensetzung nach Anspruch 9, **dadurch gekennzeichnet, dass** das Hydrolysat von *Theobroma cacao L.* Bohnen in der Zusammensetzung in einer Konzentration von 0,001 bis 20 % bezogen auf das Gesamtgewicht der Zusammensetzung, vorzugsweise von 0,1 bis 10 %, vorzugsweise von 0,2 bis 5 %, noch bevorzugter von 0,5 bis 1,5 % vorhanden ist.

11. Zusammensetzung nach einem der Ansprüche 9 oder 10, **dadurch gekennzeichnet, dass** sie zur topischen Anwendung auf der Haut formuliert ist.

12. Zusammensetzung nach einem der Ansprüche 9 bis 11 zur Verwendung zum Schutz der Haut vor den schädlichen Wirkungen von blauem Licht.

13. Zusammensetzung zur Verwendung nach Anspruch 12 zum Schutz der Haut vor oxidativem Stress, der durch Einwirkung von blauem Licht erzeugt wird.

14. Kosmetische Verwendung einer Zusammensetzung nach einem der Ansprüche 9 bis 11 zur Bekämpfung des Auftretens von Zeichen der Hautalterung und der lichtbedingten Alterung der Haut.

## Claims

1. Peptide and saccharide hydrolysate of *Theobroma cacao L.* beans, **characterised in that** the peptides and osides have a molecular weight between 200 Da and 10 kDa and **in that** said hydrolysate is obtained by the process comprising the following steps:
a) we disperse non-fermented, dried and ground *Theobroma cacao L.* beans in an aqueous phase;
b) we carry out an enzymatic treatment on the aqueous dispersion obtained in step a) in two parts, in a first treatment we use at least one carbohydrase and then in a second treatment we use at least one protease, the carbohydrase being selected from a pectinase, a cellulase, an arabanase, a hemicellulase, a xylanase, and a β-glucanase and the protease being alkaline, neutral or acidic, preferably alkaline with endopeptidase activity;
c) we denature the enzymes via a thermal treatment;
d) we recover the enzymatic hydrolysate by solid/liquid separation;
e) we purify the hydrolysate via ultrafiltration and nanofiltration, then eventually;
f) we carry out lyophilisation of the hydrolysate obtained in step e).

2. Peptide and saccharide hydrolysate of Theobroma cacao L. beans according to claim 1, **characterised in that** the beans from step a) are further delipidated.

3. Peptide and saccharide hydrolysate of *Theobroma cacao L.* beans according to claim 1 or 2, **characterised in that** the carbohydrases from step b) are a cocktail of carbohydrases comprising at least one polygalacturonase, a pectin methyl esterase, a beta glucanase, a beta mannanase, a beta glucosidase, a cellulase, a hemicellulase, an arabanase and a xylanase.

4. Peptide and saccharide hydrolysate of *Theobroma cacao L.* beans according to any one of claims 1 to 3, **characterised in that** the total concentration of enzyme used in step b) of the process is comprised between 0.1% and 3.0% by weight of the weight of the beans to be treated.

5. Peptide and saccharide hydrolysate of *Theobroma cacao L.* beans according to claim 3 or 4, **characterised in that** the carbohydrases from step b) comprise 0.5% by weight of the total weight of dry matter of cellulases and 1% by weight of the total weight of dry matter of pectinase.

6. Peptide and saccharide hydrolysate of *Theobroma cacao L.* beans according to any one of claims 1 to 5, **characterised in that** the steps a) to d) of the process are carried out at a temperature of between 20 and 90°C and a pH of between 3.0 and 11.0.

7. Peptide and saccharide hydrolysate of *Theobroma cacao L.* beans according to any one of claims 1 to 6, **characterised in that** the solid/liquid separation from step d) is performed by different processes such as centrifugation, spinning and filtration.

8. Peptide and saccharide hydrolysate of *Theobroma cacao L.* beans according to any one of claims 1 to 7, **characterised in that** at step e) ultrafiltration is carried out with a cut-off threshold of between 5 and 15 kDa and nanofiltration is carried out with a cut-off threshold of between 100 Da and 300 Da.

9. Cosmetic composition, **characterised in that** it comprises, as an active agent, an effective quantity of a hydrolysate of *Theobroma cacao L.* beans according to any one of claims 1 to 8, and a physiologically acceptable medium.

10. Composition according to claim 9, **characterised in that** the hydrolysate of *Theobroma cacao L.* beans is present in the composition at a concentration of between 0.001 to 20% in relation to the total weight of the composition, preferably from 0.1 to 10%, preferably from 0.2 to 5%, and even more preferably from 0.5 to 1.5%.

11. Composition according to claim 9 or 10, **characterised in that** it is formulated to be applied topically on the skin.

12. Composition according to any one of claims 9 to 11 for use to protect the skin from the harmful effects of blue light.

13. Composition for use according to claim 12 for protecting the skin from oxidative stress generated by exposure to blue light.

14. Cosmetic use of a composition according to any one of claims 9 to 11 for combatting the appearance of signs of ageing and photoageing of the skin.
